(19) Europäisches Patentamt | European Patent Office | Office européen des brevets

(11) **EP 4 744 691 A1**

(12) **EUROPEAN PATENT APPLICATION**
published in accordance with Art. 153(4) EPC

(43) Date of publication:
**20.05.2026 Bulletin 2026/21**

(21) Application number: **25758157.9**

(22) Date of filing: **22.01.2025**

(51) International Patent Classification (IPC):
**A61M 1/16** (2006.01)    **A61M 1/34** (2006.01)

(52) Cooperative Patent Classification (CPC):
**A61M 1/16; A61M 1/34**

(86) International application number:
**PCT/JP2025/001917**

(87) International publication number:
**WO 2025/177757 (28.08.2025 Gazette 2025/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **22.02.2024 JP 2024025784**

(71) Applicant: **Physiologas Technologies, Inc.**
**Sagamihara-shi, Kanagawa 252-0373 (JP)**

(72) Inventors:
• **MIYAWAKI, Kazuyoshi**
  **Sagamihara-shi, Kanagawa 252-0373 (JP)**
• **KOKUBO, Kenichi**
  **Sagamihara-shi, Kanagawa 252-0373 (JP)**
• **AOKI, Hiroyuki**
  **Sagamihara-shi, Kanagawa 252-0373 (JP)**

(74) Representative: **Schön, Christoph**
**Dr. Schön, Neymeyr & Partner mbB**
**Bavariaring 26**
**80336 München (DE)**

(54) **TARGET LIQUID PURIFICATION APPARATUS**

(57)    An object of the present invention is to provide a target liquid purification apparatus capable of reliably performing dialysis and removing, without excess or deficiency, a substance to be discarded during dialysis while reducing a total volume of a dialysate used in the dialysis. The target liquid purification apparatus according to the present invention includes a separation apparatus 100, an unpurified target liquid passage 11, a purified target liquid passage 12, a regenerating unit 200, a filtration dialysis effluent passage 21, a regenerated liquid passage 22, an effluent passage 24, a first replenishment liquid passage 412, a first flow rate adjustment apparatus 31, a second flow rate adjustment apparatus 32, a measurement apparatus 35 that measures a concentration of a target substance, a control apparatus 300, and a notification apparatus 400.

Fig. 1:

FIG.1

## Description

Technical Field

The present invention relates to a technique for appropriately removing toxins such as potassium ions (target substance) from dialysis effluent and the like during dialysis treatment of patients with chronic renal failure and the like.

Background Art

In hemodialysis, potassium ions and the like are removed by diffusion based on the difference in concentration between blood and dialysate in a dialysis machine by bringing the blood and the dialysate into contact with each other through a dialysis membrane. The concentration of dialysate components entering the dialysis machine is predetermined, and the dialysis effluent to which potassium ions and other toxins have been transferred from the blood is directly discarded.

While the amount of removed target substance such as potassium ions and/or the concentration of the target substance in the blood after dialysis will vary with blood flow rate and/or dialysis time, since these are treatment conditions that are primarily determined by other factors, the concentration of the target substance in the dialysate is generally varied in order to attain an appropriate amount of removed target substance and/or an appropriate concentration of the target substance in the blood after dialysis. For this reason, several dialysates with different concentrations are commercially available.

A large difference between the concentration of the target substance represented by potassium ions in the dialysate and the concentration in the patient's serum has been reported to increase the incidence of arrhythmias and/or the risk of hospitalization and death (refer to Non Patent Literature 1). Therefore, treatment preferable to the patient can be realized when the concentration of the target substance can be varied so that the difference between the concentration in the patient's serum and the concentration in the dialysate is reduced. In consideration thereof, a method has been proposed in which the concentration of potassium ions in the dialysate at the start of treatment is set 1.5 mEq/L lower than that in the patient's serum to exponentially reduce the concentration of potassium ions in the dialysate during treatment so that the difference between the concentration of potassium ions in the dialysate and the concentration in the patient's serum is reduced (refer to Non Patent Literature 2). However, since there is no apparatus for measuring the concentration in blood during dialysis and feeding back the results in order to control changes in the concentration of the dialysate, a method of treating patients while varying the concentration of dialysate to an appropriate level has not been realized.

In consideration thereof, the present inventors have invented an apparatus capable of efficiently removing a substance to be removed being a target substance such as potassium ions from a target liquid such as dialysis effluent or the like while maintaining the fluidity of the dialysis effluent or the like and controlling a difference in concentration to an appropriate level (Patent Literature 1).

Citation List

Patent Literature

Patent Literature 1: Japanese Patent Application No. 2022-132837
Patent Literature 2: U.S. Patent No. 9302038

Non Patent Literature 1: Steven M. Brunelli, David M. Spiegel, Charles Du Mond, Nina Oestreicher, Wolfgang C. Winkelmayer and Csaba P. Kovesdy. Serum-to-dialysate potassium gradient and its association with short-term outcomes in hemodialysis patients. Nephrol Dial Transplant (2018) 33:1207-1214
Non Patent Literature 2: Redaelli B, Locatelli F, Limido D et al. Effect of a new model of hemodialysis potassium removal on the control of ventricular arrhythmias. Kidney Int (1996) 50: 609-617

Summary of Invention

Technical Problem

Using the dialysis method and apparatus disclosed in Patent Literature 1 enables a target substance to be efficiently removed while controlling a difference in concentration to an appropriate level by removing unwanted ions such as potassium ion from a target liquid such as dialysis effluent or the like while maintaining the fluidity of the dialysis effluent or the like. On the other hand, when utilizing the dialysis method and apparatus disclosed in Patent Literature 1 with a conventional dialysis apparatus (for example, Patent Literature 2), since a replenishment liquid is supplied during dialysis, there is a problem in that a total dialysis volume remains high and a large amount of replenishment liquid is required and also a problem in that a total volume of dialysate cannot be reduced.

In addition, Patent Literature 1 discloses a dialysis method of removing a single target substance (only potassium ions are disclosed in an embodiment). To determine whether or not dialysis has been adequately performed, merely removing a single target substance (a substance that can be adsorbed by a single adsorbent) without excess or deficiency is insufficient, and whether or not dialysis has been adequately performed must be determined only after ensuring that a plurality of target substances have been removed without excess or deficiency. However, since Patent Literature 1 does not disclose a mode of removing a plurality of target substances without excess or deficiency, there is a problem during dialysis in that each of a plurality of target substances is not removed without excess or deficiency.

In consideration thereof, an object of the present invention is to provide a target liquid purification apparatus capable of reliably performing dialysis and removing, without excess or deficiency, a plurality of target substances to be discarded during dialysis while reducing a total volume of a dialysate used in the dialysis.

Solution to Problem

In order to achieve the object described above, the target liquid purification apparatus includes the following matters that specify the invention.

The target liquid purification apparatus is
a target liquid purification apparatus that removes at least one of a plurality of target substances from an unpurified target liquid, the target liquid purification apparatus comprising:

a separation apparatus including a porous membrane having a pore size through which the target substances contained in the unpurified target liquid can pass and a first portion and a second portion separated by the porous membrane, the separation apparatus being configured to generate a filtration dialysis effluent by introducing the unpurified target liquid containing the target substances from the first portion into the second portion while passing the unpurified target liquid through the porous membrane and to generate a purified target liquid by removing the target substances contained in the unpurified target liquid by the pass-through;
an unpurified target liquid passage configured to introduce the unpurified target liquid into the first portion of the separation apparatus;
a purified target liquid passage configured to guide the purified target liquid out from the first portion of the separation apparatus;
a filtration dialysis effluent passage configured to guide the filtration dialysis effluent out from the second portion of the separation apparatus;
an effluent passage which branches from the filtration dialysis effluent passage and which is configured to discard, among a first liquid and a second liquid as a result of the filtration dialysis effluent being separated, the first liquid as an effluent;
a regenerating unit which includes an adsorbent configured to adsorb the target substances, which is configured to remove at least one of the target substances from the second liquid by bringing the second liquid having been introduced from the filtration dialysis effluent passage in contact with the adsorbent and to generate a regenerated liquid with a reduced concentration of the target substances, and which includes two or more regenerating circuits connected in series;
a regenerated liquid passage configured to introduce the regenerated liquid from the regenerating unit into the second portion of the separation apparatus;
a first replenishment liquid passage configured to directly introduce a replenishment liquid into at least one of the unpurified target liquid passage and the purified target liquid passage; and
at least one measurement apparatus which is provided in any of the unpurified target liquid passage, the separation apparatus, and the filtration dialysis effluent passage and which is configured to measure a concentration of the target substances.

Preferably, the target liquid purification apparatus with the configuration described above comprises:

a first circuit on which one of the regenerating circuits brings an upstream-side liquid into contact with the adsorbent and then guides the liquid out to the downstream side; a second circuit on which one of the regenerating circuits introduces an upstream-side liquid into the downstream side without bringing the liquid into contact with the adsorbent; and a flow rate adjustment apparatus configured to adjust respective flow rates of the upstream-side liquid introduced into the first circuit and the second circuit, wherein
the flow rate adjustment apparatus is configured to adjust the flow rate introduced into the first circuit and the flow rate introduced into the second circuit according to a concentration of the target substances in the filtration dialysis effluent measured by the measurement apparatus.

Preferably, the target liquid purification apparatus with any of the configurations described above comprises:

an abnormality detection apparatus configured to detect an abnormality based on a time-series variation in the concentration of the target substances; and
a notification apparatus configured to notify an abnormality detected by the abnormality detection apparatus.

Brief Description of Drawings

FIG. 1 is an explanatory configuration diagram of a target liquid purification apparatus as a first embodiment.
FIG. 2 is an explanatory configuration diagram of a regenerating unit in the target liquid purification apparatus as the first embodiment.
FIG. 3 is a flow chart representing a replenishment liquid flow rate adjustment method in the target liquid purification apparatus as the first embodiment.
FIG. 4 is a flow chart representing an intra-regenerating circuit flow rate adjustment method in the target liquid purification apparatus as the first embodiment.
FIG. 5 is an explanatory configuration diagram of a target liquid purification apparatus as a second embodiment.
FIG. 6 is an explanatory configuration diagram of a target liquid purification apparatus as a third embodiment.
FIG. 7 is an explanatory configuration diagram of a target liquid purification apparatus as a fourth embodiment.
FIG. 8 is an explanatory configuration diagram of a target liquid purification apparatus as a fifth embodiment.
FIG. 9 is an explanatory configuration diagram of a target liquid purification apparatus as a sixth embodiment.
FIG. 10 is a diagram showing a concentration of urea nitrogen in an unpurified target liquid and a concentration of urea nitrogen in a first liquid for each flow rate of a replenishment liquid F41 in a target liquid purification apparatus according to an example.
FIG. 11 is a diagram showing a relationship between $Q_D$ (replenishment liquid flow rate) and $C_{DO}$ (urea nitrogen concentration in first liquid) in the target liquid purification apparatus according to the example.
FIG. 12A is a diagram showing a relationship between a urea nitrogen concentration and a position of a separation apparatus as viewed from an unpurified target liquid passage when the flow rate of the replenishment liquid is set to 500 mL/min in the target liquid purification apparatus according to the example.
FIG. 12B is a diagram showing a relationship between a urea nitrogen concentration and a position of the separation apparatus as viewed from an unpurified target liquid passage when the flow rate of the replenishment liquid is set to 230 mL/min in the target liquid purification apparatus according to the example.
FIG. 12C is a diagram showing a relationship between a urea nitrogen concentration and a position of the separation apparatus as viewed from an unpurified target liquid passage when the flow rate of the replenishment liquid is set to 150 mL/min in the target liquid purification apparatus according to the example.

Description of Embodiments

(First embodiment)

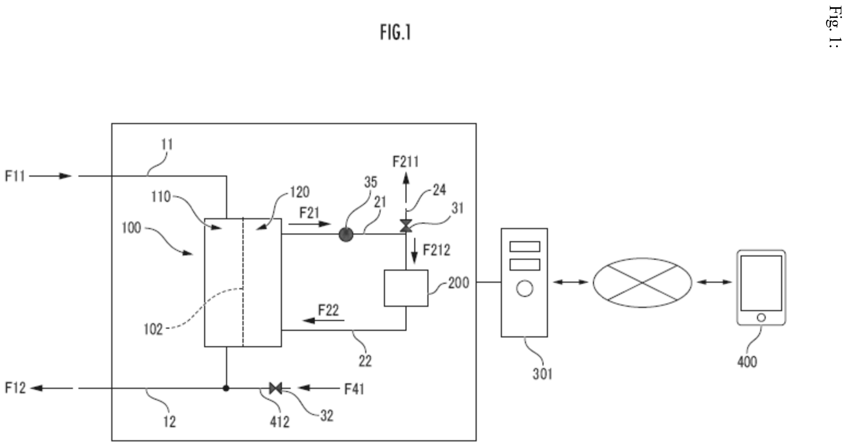

A target liquid purification apparatus as a first embodiment shown in FIG. 1 comprises a separation apparatus 100, an unpurified target liquid passage 11, a purified target liquid passage 12, a regenerating unit 200, a filtration dialysis effluent passage 21, a regenerated liquid passage 22, an effluent passage 24, a first replenishment liquid passage 412, a first flow rate adjustment apparatus 31, a second flow rate adjustment apparatus 32, a measurement apparatus 35 that measures a concentration of a target substance, a control apparatus 300, and a notification apparatus 400. Note that the first flow rate adjustment apparatus 31 and the second flow rate adjustment apparatus 32 can be omitted.

The separation apparatus 100 is divided into a first portion 110 and a second portion 120 by a porous membrane 102. The porous membrane 102 has a pore size that allows a target substance contained in an unpurified target liquid F11 (for example, blood, plasma, a dialysis effluent, or a blood filtrate) to pass through. The "target substance" is, for example, at least one ion among potassium ions, ammonium ions, calcium ions, magnesium ions, phosphate ions, hydrogen carbonate ions, and organic acid ions or a disease agent that accumulates in the body when one becomes ill such as urea, creatinine, uric acid, peptides, and proteins. The separation apparatus 100 generates a filtration dialysis effluent F21 (filtrate and/or dialysis effluent) by introducing the unpurified target liquid F11 containing the target substance from the first portion 110 into the second portion 120 by passing the unpurified target liquid F11 through the porous membrane 102. A purified target liquid F12 is generated by removing the target substance contained in the unpurified target liquid F11 by the pass-through.

The unpurified target liquid passage 11 is configured to introduce the unpurified target liquid F11 guided out from inside the body of a patient into the first portion 110 of the separation apparatus 100. The unpurified target liquid passage 11 may be provided with a flow rate adjustment apparatus (for example, a pump, a flow control valve, or a mass flow controller) (not illustrated) and may adjust the flow rate of the unpurified target liquid F11. The purified target liquid passage 12 is configured to guide the purified target liquid F12 out from the first portion 110 of the separation apparatus 100 and to introduce the purified target liquid F12 into the body of the patient. The purified target liquid passage 12 may be provided with a flow rate adjustment apparatus (for example, a pump, a flow control valve, or a mass flow controller) (not illustrated) and may adjust the flow rate of the purified target liquid F12. The separation apparatus 100 is constituted of, for example, a dialyzer. The first portion 110 is an internal space of hollow fibers constituted of a dialysis membrane. The second portion 120 is a space around the hollow fibers through which a dialysate and/or a regenerated liquid flows.

In the present embodiment, the separation apparatus 100 is configured as a "counter flow-type separation apparatus" in which a flow direction of the unpurified target liquid F11 in the first portion 110 and a flow direction of a regenerated liquid F22 in the second portion 120 oppose each other or are opposite orientations. On the other hand, in another embodiment, the separation apparatus 100 may be configured as a "parallel flow-type separation apparatus" in which the flow direction of the unpurified target liquid F11 in the first portion 110 and the flow direction of the regenerated liquid F22 in the second portion 120 are parallel to each other or are the same orientation.

The regenerating unit 200 includes an adsorbent that adsorbs the target substance. As the adsorbent, activated carbon and/or adsorbents based on activated carbon, porous adsorbents, cation exchange resins, anion exchange resins, zirconia-based ceramics, adsorbents constituted of zeolites, and mixtures thereof may be adopted. The regenerating unit 200 removes at least a part of the target substance by bringing a part of the filtration dialysis effluent (second liquid F212) into contact with the adsorbent. The regenerating unit 200 is configured to generate the regenerated liquid F22 with a reduced concentration of the target substance.

The filtration dialysis effluent passage 21 is configured to guide the filtration dialysis effluent F21 out from the second portion 120 of the separation apparatus 100 and to introduce a second liquid F212 that is a part of the filtration dialysis effluent F21 into the regenerating unit 200. The effluent passage 24 branches from the filtration dialysis effluent passage 21 and is configured to discard, among a first liquid F211 and the second liquid F212 as a result of the filtration dialysis effluent F21 being separated, the first liquid F211 as an effluent. A flow rate of the effluent in the effluent passage 24 (discarded amount of filtration dialysis effluent F21) may be appropriately controlled based on an inflow rate of a replenishment liquid (fresh dialysate) into the regenerated liquid passage 22 and/or an outflow rate of the regenerated liquid F22 from the regenerated liquid passage, taking into consideration the flow rate of the regenerated liquid F22 in the second portion 120 of the separation apparatus 100. The regenerated liquid passage 22 is configured to introduce the regenerated liquid F22 from the regenerating unit 200 into the second portion 120 of the separation apparatus 100.

The first replenishment liquid passage 412 is configured to directly introduce a first replenishment liquid F41 into the purified target liquid passage 12. For example, the "replenishment liquid" means fresh dialysate.

The first flow rate adjustment apparatus 31 is, for example, a pump, a flow control valve, or a mass flow controller and is provided in the effluent passage 24. The first flow rate adjustment apparatus 31 adjusts a flow rate of the first liquid F211 discharged from the target liquid purification apparatus during purification of the target liquid.

The second flow rate adjustment apparatus 32 is, for example, a pump, a flow control valve, or a mass flow controller and is provided in the first replenishment liquid passage 412. The second flow rate adjustment apparatus 32 adjusts a flow rate of the first replenishment liquid F41 that is fresh dialysate introduced into the first replenishment liquid passage 412 during purification of the target liquid.

As will be described later, the first flow rate adjustment apparatus 31 and the second flow rate adjustment apparatus 32 adjust flow rates according to a concentration of the target substance measured by the measurement apparatus 35. In doing so, each of the first flow rate adjustment apparatus 31 and the second flow rate adjustment apparatus 32 adjusts the flow rate so as to reduce a deviation between an introduction amount of the replenishment liquid F41 and a discharge amount of the first liquid F211 or to reduce a deviation between a total amount of the introduction amount of the replenishment liquid F41 and an amount of ultrafiltration and the discharge amount of the first liquid F212.

In this case, the "amount of ultrafiltration" refers to excess water contained in the body. The water is preferably discharged during purification of the target liquid. The amount of ultrafiltration typically varies from person to person and is calculated using a predetermined calculation method based on factors such as the user's weight, meal frequency, or height. According to the guidelines of the Japanese Society for Dialysis Therapy (Journal of the Japanese Society for Dialysis Therapy, 2013, Vol. 43, No. 7, pp. 587-632), the amount of ultrafiltration should typically be kept within 15 mL per kg of body weight per hour.

Typically, since a flow of the dialysate side tends to be uneven, the flow rate of the regenerated liquid F22 is set to approximately twice that of the unpurified target liquid F11. On the other hand, the flow rate of the regenerated liquid F22 is preferably the same as the flow rate of the unpurified target liquid F11 or lower than the flow rate of the unpurified target liquid F11. In this case, by making the flow rate of the first replenishment liquid F41 equal to the flow rate of the first liquid F211, the flow rate in the target liquid purification apparatus can be maintained constant as the flow rate of the unpurified target liquid F11. The amount of water that can be removed from the unpurified target liquid F11 is proportional to the increase in the flow rate of the first liquid F211 compared to the first replenishment liquid F41.

In addition, the flow rate of the replenishment liquid F41 and the flow rate of the first liquid F211 are approximately the same or the flow rate of the replenishment liquid F41 is smaller than the flow rate of the first liquid F211. Since the filtration dialysis effluent F21 and the regenerated liquid F22 flow within a flow path with a constant volume, when amounts of the replenishment liquid F41 and the first liquid F211 are the same, the flow rate of the unpurified target liquid F11 in the target liquid purification apparatus and the purified target liquid F12 in the target liquid purification apparatus are the same. In addition, when the replenishment liquid F41 is lower than the flow rate of the first liquid F211, the purified target liquid F12 becomes lower than the flow rate of the unpurified target liquid F11 by an amount equal to the difference between the flow rates of the replenishment liquid F41 and the first liquid F211.

At this point, by providing the measurement apparatus 35 within a passage through which the filtration dialysis effluent F21 passes, it is possible to estimate the concentration of the target substance in the unpurified target liquid F11 having to provide the measurement apparatus 35 inside a human body or within the unpurified target liquid passage 11. Since a composition of the unpurified target liquid can be determined based on a measurement result from the measurement apparatus 35, the target liquid purification system adjusts flow rates and, by extension, increases or reduces the flow rate of the replenishment liquid according to the concentration of the target substance. While the measurement apparatus 35 is preferably provided in the filtration dialysis effluent passage through which the filtration dialysis effluent F21 passes as in the mode described above, the measurement apparatus 35 is not limited thereto and one or more measurement apparatuses 35 can be provided in any of a human body, the unpurified target liquid passage, the separation apparatus, and the filtration dialysis effluent passage.

The target liquid purification system according to the present embodiment comprises the control apparatus 300 and the notification apparatus 400 as shown in FIG. 1 in addition to the target liquid purification apparatus described above. The control apparatus 300 includes: a storage device (a memory such as a RAM, a ROM, or an EEPROM, an SSD, an HDD, or the like) that stores and retains programs (software) and data; an arithmetic processing unit (a single-core processor, a multi-core processor, a CPU, or the like) that reads necessary programs and/or data from the storage device and performs predetermined arithmetic processing; an I/O circuit, and the like. As will be described later, the control apparatus 300 controls the flow rates adjusted by the first flow rate adjustment apparatus 31 and the second flow rate adjustment apparatus 32 and executes control of the target liquid purification apparatus as a whole. The control apparatus 300 is provided with an abnormality detection apparatus 301, and the abnormality detection apparatus 301 detects an abnormality according to a concentration of the target substance measured by the measurement apparatus as will be described later.

The notification apparatus 400 comprises an input interface 401 and an output interface 402. The notification apparatus 400 is a personal computer, a mobile phone (smartphone), or the like, and includes any information terminal apparatus.

(Configuration of regenerating circuit)

In the present embodiment, the regenerating unit 200 may be constituted of at least one or more regenerating circuits (regenerating circuit 201, regenerating circuit 202, ..., regenerating circuit 20"n" (where n is an integer greater than or equal to 3)). While respective regenerating circuits are preferably connected in series when there are two or more regenerating circuits, the regenerating circuits are not limited thereto and may be connected in parallel.

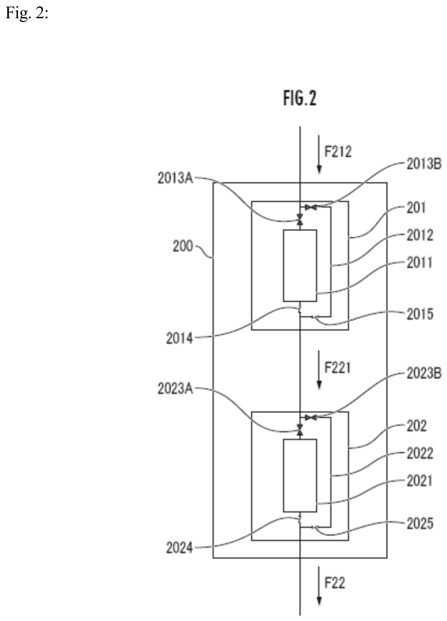

FIG. 2 schematically shows a diagram of the regenerating unit 200 being constituted of two regenerating circuits (regenerating circuit 201 and regenerating circuit 202). In this case, the regenerating circuit 201 and the regenerating circuit 202 may be mutually configured to remove the same type of target substance or may be configured to remove different types of target substances. The regenerating circuit 201 includes: a first circuit 2011 configured to bring the second liquid F212 that is an upstream-side liquid into contact with an adsorbent and then guide the liquid out to the regenerating circuit 202 that is a downstream side; a second circuit 2012 configured to guide the second liquid F212 that is an upstream-side liquid out to the downstream side without bringing the liquid into contact with the adsorbent; and a third flow rate adjustment apparatus 2013 configured to adjust respective flow rates of the second liquid F212 guided out to the first circuit 2011 and the second circuit 2012.

In addition, the regenerating circuit 201 generates a first regenerated liquid F221 and guides the first regenerated liquid F221 out to the regenerating circuit 202 which is connected in series to the regenerating circuit 201 and which is provided on the downstream side of the regenerating circuit 201. In a similar manner to the regenerating circuit 201, the regenerating circuit 202 includes: a first circuit 2021 configured to bring the first regenerated liquid F221 that is an upstream-side liquid into contact with an adsorbent and then guide the liquid out to the regenerated liquid passage 22 that is a downstream side; a second circuit 2022 configured to guide the first regenerated liquid F221 that is an upstream-side liquid out to the regenerated liquid passage 22 that is the downstream side without bringing the liquid into contact with the adsorbent; and a third flow rate adjustment apparatus 2023 configured to adjust respective flow rates of the first regenerated liquid F221 guided out to the first circuit 2021 and the second circuit 2022.

The third flow rate adjustment apparatus 2013 of the regenerating circuit 201 includes two flow control valves or flow control pumps (third flow rate adjustment apparatus 2013A and third flow rate adjustment apparatus 2013B) provided in each of the first circuit 2011 and the second circuit 2012. The flow rate adjustment apparatus provided in the first circuit 2011 and the flow rate adjustment apparatus provided in the second circuit 2012 may be the same or may differ from each other. In addition, the first circuit 2011 and the second circuit 2012 are not limited to the above and each of the first circuit 2011 or the second circuit 2021 may be configured so that by providing a flow control valve or a flow control pump in one of the first circuit 2011 and the second circuit 2021 to adjust a flow rate of a fluid flowing through one circuit and then adjust a flow rate of a fluid flowing through the other circuit, the first circuit 2011 and the second circuit 2021 adjust respective flow rates of the second liquid F212 guided out to the first circuit 2011 and the second circuit 2012. Furthermore, each of the first circuit 2011 or the second circuit 2021 may be configured so that by providing a three-way valve at a branching point between the first circuit 2011 and the second circuit 2012 to adjust a flow rate of a fluid flowing through one circuit and then adjust a flow rate of a fluid flowing through the other circuit, the first circuit 2011 and the second circuit 2012 adjust respective flow rates of the second liquid F212 guided out to the first circuit 2011 and the second circuit 2012.

In the regenerating circuit 202, while the third flow rate adjustment apparatus 2023 is configured so that the first circuit 2021 and the second circuit 2022 each includes two flow control valves or flow control pumps (third flow rate adjustment apparatus 2023A and third flow rate adjustment apparatus 2023B) in a similar manner to the regenerating circuit 201, the third flow rate adjustment apparatus 2023 is not limited thereto and may be configured in the same manner as the regenerating circuit 201. Note that the configuration of the third flow rate adjustment apparatus 2013 and the configuration of the third flow rate adjustment apparatus 2023 may be the same or may differ from each other.

The respective first circuits (first circuit 2011 and first circuit 2021) may be provided with check valves (check valve 2014 and check valve 2024) on a downstream side of the third flow rate adjustment apparatuses (third flow rate adjustment apparatus 2013 and third flow rate adjustment apparatuses 2023) in order to limit a flow rate from the downstream side to the upstream side. In addition, the respective second circuits (second circuit 2012 and second circuit 2022) may also be provided with check valves (check valve 2015 and check valve 2025) on a downstream side of the third flow rate adjustment apparatuses (third flow rate adjustment apparatus 2013 and third flow rate adjustment apparatuses 2023) in order to limit a flow rate from the downstream side to the upstream side. Providing the check valve enables dialysis to be performed efficiently in each regenerating circuit without fluid circulation. The check valve may only be provided in the first circuits (first circuit 2011 and first circuit 2021), provided in both the first circuits (first circuit 2011 and first circuit 2021) and the second circuits (second circuit 2012 and second circuit 2022), or omitted from both the first circuits (first circuit 2011 and first circuit 2021) and the second circuits (second circuit 2012 and second circuit 2022). Note that the configuration of the check valve provided in the regenerating circuit 201 and the configuration of the check valve provided in the regenerating circuit 202 may be the same or may differ from each other.

While FIG. 2 shows a configuration of an embodiment, configurations are not limited thereto and three or more regenerating circuits may be used. In this case, the regenerating circuit 202 may be configured so that after generating a second regenerated liquid F222, the regenerating circuit 202 guides the second regenerated liquid F222 out to a regenerating circuit 203 provided on a downstream side of the regenerating circuit 202. In addition, when there are four or more regenerating circuits, similar configurations may be connected in series.

While the regenerating circuit 201 and the regenerating circuit 202 are provided with the first circuits (first circuit 2011 and first circuit 2021), the second circuits (second circuit 2012 and second circuit 2022), and the third flow rate adjustment apparatuses (third flow rate adjustment apparatus 2013 and third flow rate adjustment apparatus 2023), respectively, in FIG. 2, the components may be omitted in a part of or all of the regenerating circuits. In other words, the components of the second circuit and the third flow rate adjustment apparatus may be omitted in at least one regenerating circuit of the regenerating circuit 201 and the regenerating circuit 202.

(Target liquid purification method)

According to the target liquid purification apparatus of the first embodiment with the configuration described earlier, a target liquid purification method is performed by the following procedures. Each procedure or each step may be executed in parallel. The unpurified target liquid F11 is introduced into the first portion 110 of the separation apparatus 100 (S1). The filtration dialysis effluent F21 is generated by introducing the unpurified target liquid F11 containing the target substance from the first portion 110 into the second portion 120 in the separation apparatus 100 by passing the unpurified target liquid F11 through the porous membrane 102 (S2). The purified target liquid F12 is generated by removing the target substance contained in the unpurified target liquid F11 by passing through the porous membrane 102 (S3). The purified target liquid F12 is guided out from the first portion 110 of the separation apparatus 100 (S4). The filtration dialysis effluent F21 is guided out from the second portion 120 of the separation apparatus 100 (S5). Among the first liquid F211 and the second liquid F212 as a result of the filtration dialysis effluent F21 being separated, the first liquid F211 is discarded as an effluent (S6). Due to the second liquid F212 coming into contact with the adsorbent in the regenerating unit 200, at least a part of the target substance is removed from the second liquid F212 and the regenerated liquid F22 with a reduced concentration of the target substance is generated (S7). The regenerated liquid F22 is introduced into the second portion 120 of the separation apparatus 100 (S8). Then, the first replenishment liquid F41 is directly mixed with the purified target liquid F12 (S9).

(Flow rate adjustment method in target liquid purification apparatus)

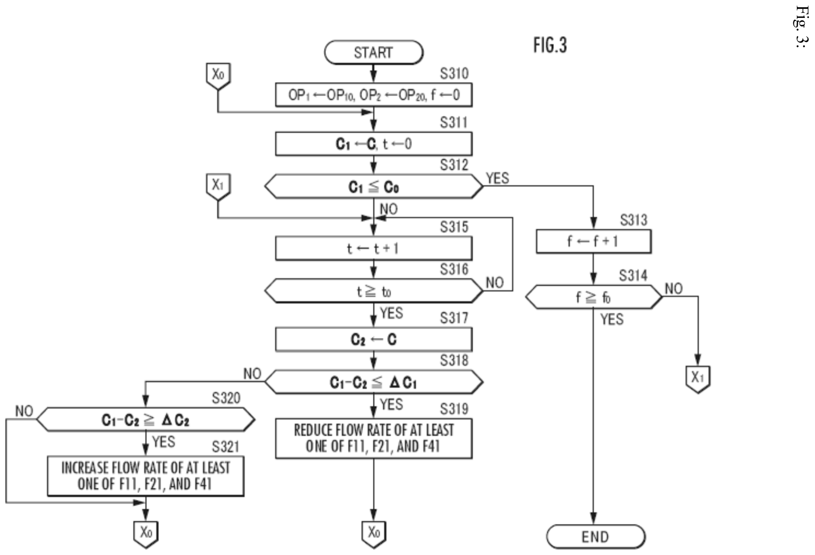

Next, a flow rate adjustment method of a replenishment liquid that is executed in the target liquid purification method described above in the target liquid purification apparatus of the first embodiment with the configuration described earlier will be described with reference to FIG. 3. First, when an operation of a target liquid purification apparatus is started in response to an ON operation of an operation switch or the like (FIG. 3/START), a degree of opening $OP_1$ of the first flow rate adjustment apparatus 31 is controlled to $OP_{10}$, a degree of opening $OP_2$ of the second flow rate adjustment apparatus 32 is controlled to $OP_{20}$, and a flag f is initialized (flag f is assigned 0) (FIG. 3/S310).

Furthermore, based on an output signal of the measurement apparatus 35, a concentration $C^{\rightarrow}$ of a substance to be removed in the filtration dialysis effluent F21 in the filtration dialysis effluent passage 21 is measured, and a current concentration $C^{\rightarrow}$ (= $(C_{10}, C_{20}, ..., C_{n0})$) of the substance to be removed is inserted into a first concentration $C_1^{\rightarrow}$ (= $(C_{11}, C_{21}, ..., C_{n1})$) that is the concentration prior to a lapse of a predetermined time (FIG. 3/S311). At this point, 0 is inserted into an elapsed dialysis time t and subsequent elapsed times are measured (FIG. 3/S311). In this case, the concentration of the target substance C is a vector including a concentration of at least one ion among potassium ions, ammonium ions, calcium ions, magnesium ions, phosphate ions, hydrogen carbonate ions, and organic acid ions or a disease agent that accumulates in the body due to renal failure and other conditions such as urea, creatinine, uric acid, peptides, and proteins. In other words, a concentration $C^{\rightarrow}$ of the target substance is a vector represented by $(C_{10}, C_{20}, ..., C_{n0})$ (where n is an integer greater than or equal to 1, and when n is 1, the concentration $C^{\rightarrow}$ of the target substance is a vector of which the number of elements is 1 and which is represented as (concentration $C^{\rightarrow}$ = $(C_{10})$)).

Next, whether or not the first concentration $C_1^{\rightarrow}$ (= $(C_{11}, C_{21}, ..., C_{n1})$) is equal to or lower than a designated concentration $C_0^{\rightarrow}$ (= $(C_{100}, C_{200}, ..., C_{n00})$) is determined (FIG. 3/S312). The "designated concentration" refers to a concentration indicating that the target substance is sufficiently within normal levels. The value of the designated concentration is also important from the perspective of adverse effects on human health when the concentration of the target substance in the target liquid is extremely low. The "designated concentration" is defined for each element of the target substance $C^{\rightarrow}$ (the concentration of each target substance). At this point, each element of the specified concentration $C_0^{\rightarrow}$ is different. For example, while the target liquid includes at least one ion among potassium ions, ammonium ions, calcium ions, magnesium ions, phosphate ions, hydrogen carbonate ions, and organic acid ions or a disease agent that accumulates in the body due to renal failure and other conditions such as urea, creatinine, uric acid, peptides, and proteins, potassium ions may possibly decrease more than necessary compared to other target substances. In this case, reducing potassium ion concentration beyond what is necessary can cause muscle weakness, cramps, or spasms, and even paralysis, as well as potentially induce arrhythmia. Therefore, an individual designated concentration is preferably defined for each target substance.

The designated concentration $C_0^{\rightarrow}$ may vary according to target liquid purification time. In other words, the designated concentration $C_0^{\rightarrow}$ immediately after the start of target liquid purification may differ from the designated concentration $C_0^{\rightarrow}$ immediately before the end of target liquid purification. In particular, the designated concentration $C_0^{\rightarrow}$ is preferably set to as to decrease continuously or discontinuously over time. In addition, each element ($C_{100}, C_{200}, ..., C_{n00}$) of the designated concentration $C_0^{\rightarrow}$ may change independently in response to the target liquid purification time or may change in relation to each other in response to the target liquid purification time.

Furthermore, it is undesirable to continue dialysis when the concentration of one target substance is equal to or below the designated concentration. Therefore, when the concentration of a target substance is below the designated concentration, even if the dialysis time is short, dialysis must be terminated or the concentration of the target substance should be prevented from decreasing beyond what is necessary. To this end, one target substance is prevented from decreasing beyond what is necessary by, after completing dialysis, changing the configuration of the regenerating unit 200, the replenishment liquid F41, or the like and then restarting dialysis. Alternatively, as will be described later, a plurality of regenerating circuits are used to prevent the concentration of one target substance from decreasing beyond what is necessary.

The determination of whether the first concentration $C_1^{\rightarrow}$ is equal to or lower than the designated concentration $C_0^{\rightarrow}$ may be made for each element of the vector, and when all of the elements are affirmative, the first concentration $C_1^{\rightarrow}$

may be determined to be equal to or lower than the designated concentration $C_0^{\rightarrow}$. When a part of the elements (concentration of a target substance that affects the human body when it decreases beyond what is necessary) is affirmative, the determination may involve determining that the first concentration $C_1^{\rightarrow}$ is equal to or lower than the designated concentration $C_0^{\rightarrow}$. When a magnitude of the first concentration $C_1^{\rightarrow}$ is equal to or smaller than a magnitude of the designated concentration $C_0^{\rightarrow}$, the determination may involve determining that the first concentration $C_1^{\rightarrow}$ is equal to or lower than the designated concentration $C_0^{\rightarrow}$. In addition, when a magnitude of a difference between a deviation of the first concentration $C_1^{\rightarrow}$ and a deviation of the designated concentration $C_0^{\rightarrow}$ is determined to be equal to or smaller than a reference value, the first concentration $C_1^{\rightarrow}$ may be determined to be equal to or lower than the designated concentration $C_0^{\rightarrow}$. Furthermore, when deviations of a part of the elements are weighted and a magnitude of a deviation between the first concentration $C_1^{\rightarrow}$ and the designated concentration $C_0^{\rightarrow}$ is determined to be equal to or smaller than a reference value, the first concentration $C_1^{\rightarrow}$ may be determined to be equal to or lower than the designated concentration $C_0^{\rightarrow}$. In this case, a magnitude refers to concepts such as the norm of the vector and examples thereof include a Euclidean distance, a Chebyshev distance, or a Manhattan distance from an origin or a Euclidean distance, a Chebyshev distance, or a Manhattan distance between vectors.

In addition, the determination may be performed using any machine learning based on a part of or all of the elements of $C_1^{\rightarrow}$. The machine learning may involve any supervised learning using training data including judgments of medical professionals such as physicians in past target liquid purification treatments.

When the determination result is positive (FIG. 3/S312...YES), the flag f is increased by 1 (FIG. 3/S313). Next, whether or not the flag f is equal to or larger than a threshold $f_0$ is determined (FIG. 3/S314). At the start of dialysis, a dialysate may remain in a dialyzer, potentially causing the first concentration $C_1$ to be lower than the concentration of the target substance in the blood. Therefore, when the first concentration is once again lower than the designated concentration $C_0$ after the predetermined time $t_0$ has elapsed, a dialysis end operation is to be performed.

When the determination result is positive (FIG. 3/S314...YES), the target liquid purification apparatus ends the purification of the target liquid (FIG. 3/END). At this point, ultrafiltration that involves removing excess water accumulated within the body is performed by making the degree of opening $OP_1$ of the first flow rate adjustment apparatus 31 larger than the degree of opening $OP_2$ of the second flow rate adjustment apparatus 32. Note that in the present embodiment, ultrafiltration may be performed uniformly during the purification of the target liquid or may be varied according to the elapsed time during the purification of the target liquid. For example, ultrafiltration may involve varying a hematocrit value over time relative to a reference. In other words, ultrafiltration is preferably performed in a manner that minimizes variations in blood volume as much as possible.

On the other hand, when the determination result is negative (FIG. 3/S314...NO), processing of connector $X_1$ and thereafter is executed. Accordingly, a dialysis operation can be controlled to reliably remove the target substance even when the concentration of the target substance is low during the initial stages of dialysis. Then, processing of S315 and thereafter to be described later is executed. Note that processing of S313 to S314 may be omitted in the present embodiment.

On the other hand, when the determination result in S312 is negative (FIG. 3/S312...NO), the target liquid purification apparatus determines that purification of the target liquid is insufficient and the target liquid purification operation is continued. Next, t is increased by 1 (FIG. 3/S316). Note that, at this point, an elapsed time from a stage where 0 is inserted to t (FIG. 3/S311) may be automatically measured and a transition to S316 may be performed after the automatic measurement.

Subsequently, whether or not an elapsed dialysis time t has exceeded a predetermined elapsed time $t_0$ is determined (FIG. 3/S316). When the determination result is negative (FIG. 3/S316...NO), processing of S315 and thereafter is executed once again.

When the determination result is positive (FIG. 3/S316...YES), a current concentration C of the substance to be removed is inserted into a second concentration $C_2^{\rightarrow}$ (= $(C_{12}, C_{22}, ..., C_{n2})$) that is a concentration after the lapse of a predetermined time (FIG. 3/S317).

Next, whether or not a difference between the first concentration $C_1^{\rightarrow}$ and the second concentration $C_2^{\rightarrow}$, $C_1^{\rightarrow}$ - $C_2^{\rightarrow}$ (= $(C_{11} - C_{12}, C_{21} - C_{22}, ..., C_{n1} - C_{n2})$) is equal to or lower than a first concentration difference threshold $\Delta C_1^{\rightarrow}$ (= $(\Delta C_{11}, \Delta C_{12}, ..., \Delta C_{12})$) is determined (FIG. 3/S318). The first concentration difference threshold $\Delta C_1^{\rightarrow}$ is a threshold for determining whether sufficient dialysis has been performed after a lapse of a predetermined elapsed time $t_0$, and the first concentration difference threshold $\Delta C_1^{\rightarrow}$ that forms the basis of the determination processing may be adopted in consideration of the predetermined elapsed time $t_0$. A magnitude of the concentration difference $C_1^{\rightarrow}$ - $C_2^{\rightarrow}$ represents a high or low removal performance (or adsorption performance) of the substance to be removed by the target liquid purification apparatus. Note that the first concentration difference threshold $\Delta C_1^{\rightarrow}$ is a vector having respective thresholds for each target substance as elements, and each element typically has a different value. In addition, a part of or all of the elements of the first concentration difference threshold $\Delta C_1^{\rightarrow}$ that forms the basis of the determination processing may be adopted in consideration of the predetermined elapsed time $t_0$. The first concentration difference threshold $\Delta C_1^{\rightarrow}$ may vary according to target liquid purification time. In other words, the first concentration difference threshold $\Delta C_1^{\rightarrow}$ immediately

after the start of target liquid purification may differ from the first concentration difference threshold $\Delta C_1^\rightarrow$ immediately before the end of target liquid purification. In particular, the first concentration difference threshold $\Delta C_1^\rightarrow$ is preferably set to as to decrease continuously or discontinuously over time. In addition, each element ($\Delta C_{11}$, $\Delta C_{12}$, ..., $\Delta C_{12}$) of the first concentration difference threshold $\Delta C_1^\rightarrow$ may change independently in response to the target liquid purification time or may change in relation to each other in response to the target liquid purification time.

The determination of whether or not $C_1^\rightarrow - C_2^\rightarrow$ is equal to or lower than the first concentration difference threshold $\Delta C_1^\rightarrow$ may involve performing processing similar to the determination in S312 described earlier. Therefore, a description will be omitted from the perspective of duplication. On the other hand, the determination in S318 may involve performing processing by the same processing method as S311 or performing processing by a different processing method.

When the determination result is positive (FIG. 3/S318... YES), the target liquid purification apparatus is controlled so that the flow rate of at least one of the replenishment liquid F41, the unpurified target liquid F11, and the filtration dialysis effluent F21 is reduced (FIG. 3/S319). The control is executed after controlling, for example, output of a flow rate adjustment apparatus (pump) provided in at least one passage of the unpurified target liquid passage 11, the filtration dialysis effluent passage 21, and a replenishment liquid passage 41. In addition, the control may be executed after controlling operations of the first flow rate adjustment apparatus 31 and the second flow rate adjustment apparatus 32. Accordingly, the flow rate of the replenishment liquid F41 can be limited. Then, processing of connector $X_0$ and thereafter is executed. In other words, measurement processing for initializing the first concentration $C_1^\rightarrow$ and the dialysis start time t (FIG. 3/S311) and thereafter is repeated.

A degree of decrease of the flow rate of at least one of the replenishment liquid F41, the unpurified target liquid F11, and the filtration dialysis effluent F21 may be adjusted in consideration of a magnitude of the concentration difference $C_1^\rightarrow - C_2^\rightarrow$ or a magnitude of the predetermined elapsed time $t_0$. Accordingly, an amount of the replenishment liquid F41 can be optimized. In this case, a minimum value of the flow rate of at least one of the replenishment liquid F41, the unpurified target liquid F11, and the filtration dialysis effluent F21 may be provided, and when the flow rate falls below the minimum value in S319, processing of assigning the minimum value to the flow rate may be performed.

On the other hand, when the determination result is negative (FIG. 3/S318...NO), whether or not the difference between the first concentration $C_1^\rightarrow$ and the second concentration $C_2^\rightarrow$, $C_1^\rightarrow - C_2^\rightarrow$, is equal to or greater than a second concentration difference threshold $\Delta C_2^\rightarrow$ (= ($\Delta C_{21}$, $\Delta C_{22}$, ..., $\Delta C_{22}$)) is determined (FIG. 3/S320). The second concentration difference threshold $\Delta C_2^\rightarrow$ is a threshold for determining whether dialysis after a lapse of the predetermined elapsed time $t_0$ is insufficient, and a part of or all of elements of the second concentration difference threshold $\Delta C_2^\rightarrow$ that forms the basis of the determination processing may be adopted in consideration of the predetermined elapsed time $t_0$. The second concentration difference threshold $\Delta C_2^\rightarrow$ may vary according to target liquid purification time. In other words, the second concentration difference threshold $\Delta C_2^\rightarrow$ immediately after the start of target liquid purification may differ from the second concentration difference threshold $\Delta C_2^\rightarrow$ immediately before the end of target liquid purification. In particular, the second concentration difference threshold $\Delta C_2^\rightarrow$ is preferably set to as to decrease continuously or discontinuously over time. In addition, each element ($\Delta C_{21}$, $\Delta C_{22}$, ..., $\Delta C_{22}$) of the second concentration difference threshold $\Delta C_2^\rightarrow$ may change independently in response to the target liquid purification time or may change in relation to each other in response to the target liquid purification time.

The determination of whether or not $C_1^\rightarrow - C_2^\rightarrow$ is equal to or higher than the second concentration difference threshold $\Delta C_2^\rightarrow$ may involve performing processing similar to the determination in S312 described earlier. Note that a description of the determination processing will be omitted from the perspective of duplication. On the other hand, the determination in S320 may involve performing processing by the same processing method as one of S311 and S318 or performing processing by a different processing method from both S311 and S318.

When the determination result is positive (FIG. 3/S320...YES), the target liquid purification apparatus is controlled so that the flow rate of at least one of the replenishment liquid F41, the unpurified target liquid F11, and the filtration dialysis effluent F21 is increased (FIG. 3/S321). The control is executed after controlling, for example, output of a flow rate adjustment apparatus (pump) provided in at least one passage of the unpurified target liquid passage 11, the filtration dialysis effluent passage 21, and the replenishment liquid passage 41. In addition, the control may be executed after controlling operations of the first flow rate adjustment apparatus 31 and the second flow rate adjustment apparatus 32. Accordingly, a dialysis operation can be controlled to reliably remove the target substance even when dialysis is insufficient. Then, processing of connector $X_0$ and thereafter is executed. In other words, measurement processing for initializing the first concentration $C_1^\rightarrow$ and the dialysis start time t (FIG. 3/S312) and thereafter is repeated. Note that processing of S320 to S321 may be omitted in the present embodiment.

On the other hand, when the determination result is negative (FIG. 3/S320...NO), processing of connector $X_0$ and thereafter is executed without controlling the operations of the first flow rate adjustment apparatus 31 and the second flow rate adjustment apparatus 32. In other words, measurement processing for initializing the first concentration $C_1^\rightarrow$ and the dialysis start time t (FIG. 3/S312) and thereafter is repeated. Accordingly, dialysis operations are controlled to prevent the unintended removal of target substances by dialysis.

While the predetermined elapsed time $t_0$, the degree of increase or decrease of the flow rate of at least one of the replenishment liquid F41, the unpurified target liquid F11, and the filtration dialysis effluent F21, the first concentration difference threshold $\Delta C_1^{\rightarrow}$, and the second concentration difference threshold $\Delta C_2^{\rightarrow}$ are constant in the replenishment liquid flow rate adjustment method in the embodiment described above, the values may vary according to the number of times the predetermined elapsed time $t_0$ has elapsed or the first concentration $C_1^{\rightarrow}$. Accordingly, excess target substances can be reliably removed while optimizing an amount of the replenishment liquid F41 and preventing the target substances from significantly decreasing.

While the flow rate of at least one of the replenishment liquid F41, the unpurified target liquid F11, and the filtration dialysis effluent F21 is adjusted when $C_1^{\rightarrow} - C_2^{\rightarrow}$ is equal to or smaller than a predetermined value in S318 to S321, the adjustment of flow rates is not limited to the embodiment described above and the flow rate of at least one of the replenishment liquid F41, the unpurified target liquid F11, and the filtration dialysis effluent F21 may be adjusted based on any function that uses a part of or all of the elements of $C_1^{\rightarrow} - C_2^{\rightarrow}$ as variables. Note that in the function, a part of the variables may be weighted. In other words, S318 to S321 after S317 may be omitted, a function where each of the elements of $C_1^{\rightarrow} - C_2^{\rightarrow}$ is a variable may be calculated for each of the degree of increase or decrease of the flow rate of at least one of the replenishment liquid F41, the unpurified target liquid F11, and the filtration dialysis effluent F21, and processing of connector $X_0$ and thereafter may be executed.

In addition, the flow rate of at least one of the replenishment liquid F41, the unpurified target liquid F11, and the filtration dialysis effluent F21 may be adjusted based on any function that uses a part of or all of the elements of the second concentration $C_2^{\rightarrow}$ as variables. Note that in the function, a part of the variables may be weighted. In other words, S318 to S321 after S317 may be omitted, a function where each of the elements of the second concentration $C_2^{\rightarrow}$ is a variable may be calculated for the flow rate of at least one of the replenishment liquid F41, the unpurified target liquid F11, and the filtration dialysis effluent F21, and processing of connector $X_0$ and thereafter may be executed.

The flow rate adjustment described above may be performed based on any machine learning based on a part of or all of the elements of $C_1^{\rightarrow} - C_2^{\rightarrow}$. The machine learning may involve any supervised learning using judgments of medical professionals such as physicians in past target liquid purification treatments as training data.

In addition, while the first embodiment has been described as an example in the embodiment described above, otherwise, for example, even when the replenishment liquid is provided by a plurality of passages as in second to sixth embodiments to be described later, the flow rate adjustment method in the target liquid purification apparatus described above may be performed by independently adjusting each replenishment liquid flow path.

(Intra-regenerating circuit flow rate adjustment method)

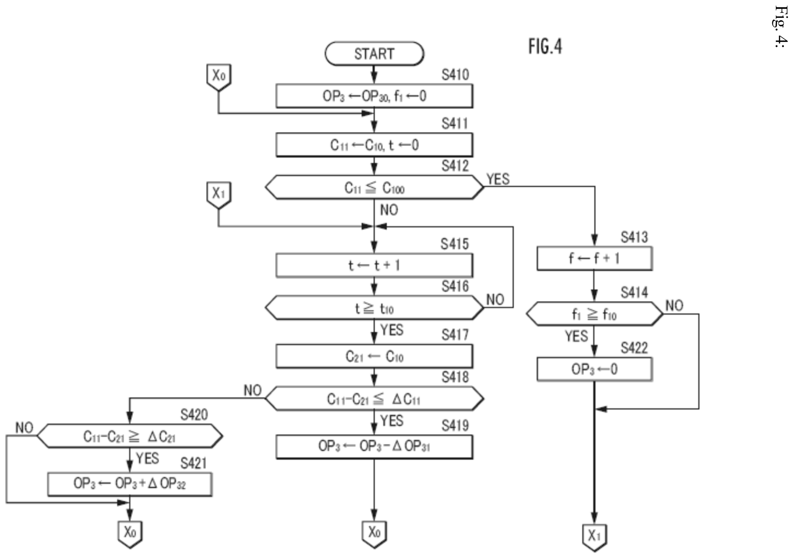

Next, a flow rate adjustment method in the third flow rate adjustment apparatus 2013 in the regenerating circuit 201 that is executed in the target liquid purification method described above in the target liquid purification apparatus of the first embodiment with the configuration described earlier will be described with reference to FIG. 4. While reference sign "S4OO" will be assigned in the processing, this means that "S4OO" is performed in parallel with "S3OO" or performed immediately before or after "S3OO" in the processing described earlier (meaning that the same numerals (10 to 21) are to be inserted into each "OO"). In other words, the processing in "S410" is performed parallel with "S310" or performed immediately before or after "S310". Note that the processing in FIG. 4 may be executed by omitting all of or a part of S310 to S321 in FIG. 3.

In the present embodiment, an intra-regenerating circuit flow rate adjustment method will be described using the regenerating circuit 201 as an example. In this case, while the regenerating circuit 201 is a regenerating circuit containing an adsorbent that adsorbs potassium ions, it is obvious that the regenerating circuit 201 is not limited thereto and may be based on any target substance.

In the processing, first, when an operation of the target liquid purification apparatus is started in response to an ON operation of an operation switch or the like (FIG. 4/START), a degree of opening $OP_3$ of the third flow rate adjustment apparatus 2013 is controlled to $OP_{30}$ and a flag $f_1$ is initialized (flag $f_1$ is assigned 0) (FIG. 4/S410). In this case, a state where $OP_3$ is 0 means that the second liquid is only introduced into the second circuit.

Furthermore, based on an output signal of the measurement apparatus 35, a concentration $C_{10}$ of any substance to be removed (potassium ions) in the filtration dialysis effluent F21 in the filtration dialysis effluent passage 21 is measured. Note that a first concentration $C_{11}$ of potassium ions is one of the elements of the first concentration $C_1^{\rightarrow}$. The current concentration $C_{10}$ of the potassium ions is inserted into the first concentration $C_{11}$ of potassium ions that is a concentration before the lapse of a predetermined time (FIG. 4/S411). At this point, 0 is inserted into the elapsed dialysis time t and subsequent elapsed times are measured (FIG. 4/S411).

Next, whether or not the first concentration $C_{11}$ of potassium ions is equal to or lower than a designated concentration $C_{100}$ of potassium ions is determined (FIG. 4/S412). The "designated concentration" refers to a value indicating that the concentration of the target substance is sufficiently within normal levels. The value of the designated concentration is also important from the perspective of adverse effects on human health when the concentration of the target substance in the target liquid is extremely low. The designated concentration $C_{100}$ may vary according to target liquid purification time. In other words, the designated concentration $C_{100}$ immediately after the start of target liquid purification may differ from the designated concentration $C_{100}$ immediately before the end of target liquid purification. In particular, the designated concentration $C_{100}$ is preferably set to as to decrease continuously or discontinuously over time.

When the determination result is positive (FIG. 4/S412...YES), the flag $f_1$ is increased by 1 (FIG. 4/S413). Next, whether or not the flag $f_1$ is equal to or larger than a threshold $f_{10}$ is determined (FIG. 4/S414). At the start of dialysis, a dialysate may remain in a dialyzer, potentially causing the first concentration $C_{11}$ of potassium ions to be lower than the concentration of the target substance in the blood. Therefore, if a situation where the first concentration $C_{11}$ of potassium ions once again falls below the designated concentration $C_{10}$ of potassium ions after the lapse of a predetermined time $t_{10}$ occurs repeatedly a plurality of times, control is performed to prevent excessive removal of potassium ions.

When the determination result is positive (FIG. 4/S414...YES), 0 is inserted to the degree of opening $OP_3$ of the third flow rate adjustment apparatus 2013. Note that a state where $OP_3$ is 0 means that the second liquid F212 is only introduced into the second circuit 2012. Therefore, the target liquid purification treatment can be performed so as to reduce other target substances without inadvertently reducing potassium ions.

On the other hand, when the determination result is negative (FIG. 3/S414...NO), processing of connector $X_1$ and thereafter is executed. Accordingly, the dialysis operation is controlled to reliably remove the target substance despite a low concentration of potassium ions due to dilution by a priming solution or the like during the initial stages of dialysis. Then, processing of S415 and thereafter to be described later is executed. Note that processing of S413 to S414 and S412 may be omitted in the present embodiment.

On the other hand, when the determination result in S412 is negative (FIG. 4/S412...NO), the target liquid purification apparatus determines that purification of potassium ions in the target liquid is insufficient and the target liquid purification operation is continued. Next, t is increased by 1 (FIG. 4/S416). At this point, an elapsed time from a stage where 0 is inserted to t (FIG. 4/S411) may be automatically measured and a transition to S416 may be performed after the automatic measurement.

Subsequently, whether or not an elapsed dialysis time t has exceeded a predetermined elapsed time $t_{10}$ is determined (FIG. 4/S416). When the determination result is negative (FIG. 4/S416...NO), processing of S415 and thereafter is executed once again. Note that $t_{10}$ and $t_0$ may be the same or may differ from each other.

When the determination result is positive (FIG. 4/S416...YES), the current concentration $C_{10}$ of the potassium ions is inserted into the second concentration $C_{21}$ of potassium ions that is a concentration after the lapse of a predetermined time (FIG. 4/S417).

Next, whether or not a difference between the first concentration $C_{11}$ and the second concentration $C_{21}$ of potassium ions, $C_{11} - C_{21}$, is equal to or lower than a first concentration difference threshold $\Delta C_{11}$ of potassium ions is determined (FIG. 4/S418). The first concentration difference threshold $\Delta C_{11}$ of potassium ions is a threshold for determining whether sufficient dialysis has been performed after a lapse of a predetermined elapsed time $t_{10}$, and the first concentration difference threshold $\Delta C_{11}$ that forms the basis of the determination processing may be adopted in consideration of the predetermined elapsed time $t_{10}$. A magnitude of the concentration difference $C_{11} - C_{21}$ represents a high or low removal performance (or adsorption performance) of potassium ions by the target liquid purification apparatus. The first concentration difference threshold $\Delta C_{11}$ may vary according to target liquid purification time. In other words, the first concentration difference threshold $\Delta C_{11}$ immediately after the start of target liquid purification may differ from the first concentration difference threshold $\Delta C_{11}$ immediately before the end of target liquid purification. In particular, the first concentration difference threshold $\Delta C_{11}$ is preferably set to as to decrease continuously or discontinuously over time.

When the determination result is positive (FIG. 4/S418...YES), the third flow rate adjustment apparatus 2013 is controlled so that the degree of opening $OP_3$ of the third flow rate adjustment apparatus 2013 is lowered by $\Delta OP_{31}$ (FIG. 4/S419). In this case, a minimum value of the degree of opening $OP_3$ is 0, and when the degree of opening $OP_3$ is negative in S319, 0 is assigned to the degree of opening $OP_3$. Accordingly, an excessive reduction of potassium ions from body fluids can be prevented. In other words, measurement processing for initializing the first concentration $C_{11}$ and the dialysis start time t (FIG. 4/S411) and thereafter is repeated.

The third flow rate adjustment apparatus 2013 may be adjusted in consideration of a magnitude of the concentration difference $C_{11} - C_{21}$ or a magnitude of the predetermined elapsed time $t_{10}$. Accordingly, an excessively rapid decrease in potassium ion concentration can be reduced.

On the other hand, when the determination result is negative (FIG. 4/ S418...NO), whether or not the difference between the first concentration $C_{11}$ and the second concentration $C_{21}$, $C_{11} - C_{21}$, is equal to or greater than a second concentration difference threshold $\Delta C_{21}$ of potassium ion is determined (FIG. 4/S420). The second concentration difference threshold $\Delta C_{21}$ of potassium ions is a threshold for determining whether dialysis after a lapse of the predetermined elapsed time $t_{10}$ is insufficient, and the second concentration difference threshold $\Delta C_{21}$ that forms the basis of the determination processing may be adopted in consideration of the predetermined elapsed time $t_{10}$. The second concentration difference threshold $\Delta C_{21}$ may vary according to target liquid purification time. In other words, the second concentration difference threshold $\Delta C_{21}$ immediately after the start of target liquid purification may differ from the second

concentration difference threshold $\Delta C_{21}$ immediately before the end of target liquid purification. In particular, the second concentration difference threshold $\Delta C_{21}$ is preferably set to as to decrease continuously or discontinuously over time.

When the determination result is positive (FIG. 4/S420...YES), the operation of the third flow rate adjustment apparatus 2013 is controlled so that the degree of opening $OP_3$ of the third flow rate adjustment apparatus 2013 is increased by $\Delta OP_{32}$ (FIG. 4/S421). Accordingly, a dialysis operation can be controlled to reliably remove potassium ions even if adsorption of the potassium ions is insufficient. Then, processing of connector $X_0$ and thereafter is executed. In other words, measurement processing for initializing the first concentration $C_{11}$ and the dialysis start time t (FIG. 4/S412) and thereafter is repeated. Note that processing of S420 to S421 may be omitted in the present embodiment.

On the other hand, when the determination result is negative (FIG. 4/S420...NO), processing of connector $X_0$ and thereafter is executed without controlling the operations of the third flow rate adjustment apparatus 2013. In other words, measurement processing for initializing the first concentration $C_{11}$ and the dialysis start time t (FIG. 4/S412) and thereafter is repeated. Accordingly, dialysis operations are controlled to prevent the unintended removal of target substances by dialysis.

While the predetermined elapsed time $t_{10}$, a degree of opening variation $\Delta OP_{31}$, a degree of opening variation $\Delta OP_{32}$, the first concentration difference threshold $\Delta C_{11}$, and the second concentration difference threshold $\Delta C_{21}$ are constant in the intra-regenerating circuit flow rate adjustment method according to the embodiment described above, the values may vary according to the number of times the predetermined elapsed time $t_{10}$ has elapsed or the first concentration $C_{11}$. Accordingly, while potassium ions can be removed from body fluids certainly, an excessive decrease of the concentration of potassium ions can be prevented.

In FIG. 2, since control similar to the flow rate adjustment in the regenerating circuit 201 described above is performed in the regenerating circuit 202 that is provided on the downstream side of the regenerating circuit 201, a description of the control will be omitted. Note that the adsorbent provided in the regenerating circuit 202 is an adsorbent that adsorbs a different target substance from the adsorbent provided in the regenerating circuit 201 and is, for example, an adsorbent that adsorbs urea nitrogen. In this case, the control in the regenerating circuit 201 and the control in the regenerating circuit 202 are controlled independently of each other. Additionally, three or more regenerating circuits may be provided, and in this case as well, each is controlled independently of the others. Accordingly, for example, even when potassium ions decrease excessively while urea nitrogen remains present, urea nitrogen can be efficiently removed while suppressing the removal of potassium ions.

While the degree of opening $OP_3$ is adjusted solely based on the concentration of $C_{11}$ - $C_{21}$ in S418 to S421, the adjustment of the degree of opening $OP_3$ is not limited to the embodiment described above and the degree of opening $OP_3$ may be adjusted based on any function that uses $C_{11}$ - $C_{21}$ as a variable. In other words, S418 to S421 after S417 may be omitted, the degree of opening $OP_3$ may be adjusted as $OP_3 = OP_3 (C_{11} - C_{21})$, and processing of connector $X_0$ and thereafter may be executed.

In addition, the degree of opening $OP_3$ may be adjusted based on any function that uses $C_{21}$ as a variable. In other words, S418 to S421 after S417 may be omitted, the degree of opening $OP_3$ may be adjusted as $OP_3 = OP_3 (C_{11} - C_{21})$, and processing of connector $X_0$ and thereafter may be executed.

While the processing described in FIG. 3 (S310 to S321) and the processing described in FIG. 4 (S410 to S422) are performed under the code "S4OO" in parallel with "S3OO" or immediately before or after "S3OO" in the present embodiment, processing is not limited thereto. In the present embodiment, S410 to S422 may be omitted and S310 to S321 may be performed in the processing (S310 to S321) described in FIG. 3, or S310 to S321 may be omitted and S410 to S422 may be performed in the processing (S410 to S422) described in FIG. 4. When S310 to S321 is omitted, the processing (S410 to S422) described in FIG. 4 may be ended once a predetermined time elapses.

(Second embodiment)

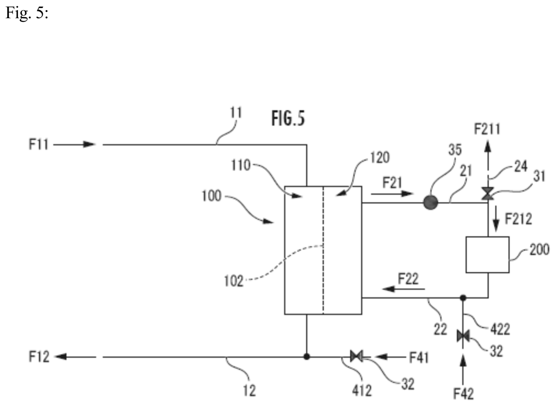

A target liquid purification apparatus as a second embodiment shown in FIG. 5 has a configuration in which a second replenishment liquid passage 422 has been added to the target liquid purification apparatus (refer to FIG. 1) according to the first embodiment. The second replenishment liquid passage 422 is configured to directly introduce a second replenishment liquid F42 into the regenerated liquid passage 22. Supply sources of the second replenishment liquid F42 and the first replenishment liquid F41 may be a common supply source or separate supply sources.

At this point, a total flow rate of the second replenishment liquid F42 and the first replenishment liquid F41 is preferably approximately equal to the flow rate of the first liquid F211 and, in the replenishment liquid flow rate adjustment method, a total flow rate of the second replenishment liquid F42 and the first replenishment liquid F41 is controlled so as to be approximately equal to the flow rate of the first liquid F211.

Flow rates of the second replenishment liquid F42 and the first replenishment liquid F41 may be the same or may differ from each other. For example, when the ion concentration detected by the measurement apparatus 35 becomes the same as that of the unpurified target liquid F11, the flow rates of the second replenishment liquid F42 and the first replenishment liquid F41 can be adjusted based on the membrane's susceptibility to clogging, removal characteristics of substances in the unpurified target liquid F11 within the separation apparatus 100, and the like.

Since other components of the target liquid purification apparatus and the replenishment liquid flow rate adjustment method according to the second embodiment are substantially similar to those of the target liquid purification apparatus and the replenishment liquid flow rate adjustment method according to the first embodiment, similar components will be assigned the same reference signs and descriptions thereof will not be repeated.

(Third embodiment)

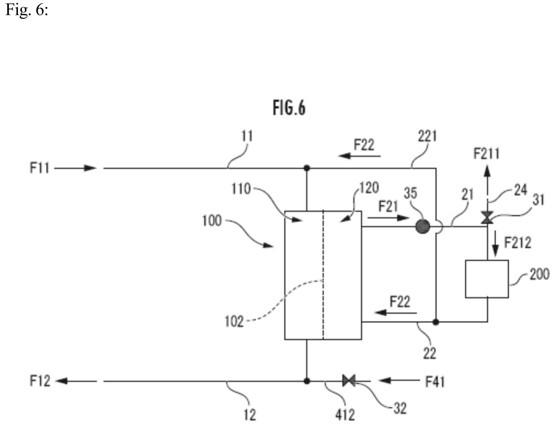

A target liquid purification apparatus as a third embodiment shown in FIG. 6 has a configuration in which a regenerated liquid branch passage 221 has been added to the target liquid purification apparatus (refer to FIG. 1) according to the first embodiment. The regenerated liquid branch passage 221 is configured to directly introduce a part of the regenerated liquid F22 into the unpurified target liquid passage 11.

Since other components of the target liquid purification apparatus and the replenishment liquid flow rate adjustment method according to the third embodiment are substantially similar to those of the target liquid purification apparatus and the replenishment liquid flow rate adjustment method according to the first embodiment, similar components will be assigned the same reference signs and descriptions thereof will not be repeated.

(Fourth embodiment)

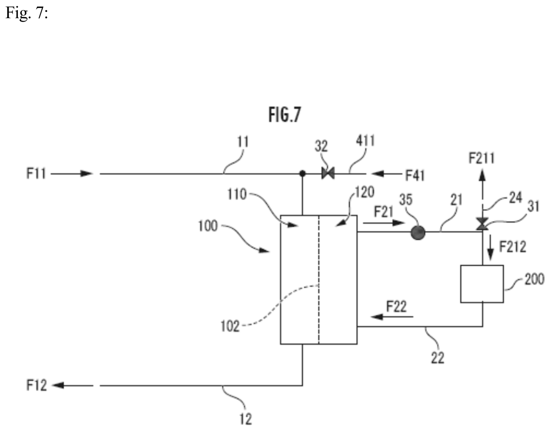

A target liquid purification apparatus as a fourth embodiment shown in FIG. 7 comprises a first replenishment liquid passage 411 in place of the first replenishment liquid passage 412 in the target liquid purification apparatus (refer to FIG. 1) according to the first embodiment. The first replenishment liquid passage 411 is configured to directly introduce a first replenishment liquid F41 into the unpurified target liquid passage 11 instead of the purified target liquid passage 12.

Since other components of the target liquid purification apparatus and the replenishment liquid flow rate adjustment method according to the fourth embodiment are substantially similar to those of the target liquid purification apparatus and the replenishment liquid flow rate adjustment method according to the first embodiment, similar components will be assigned the same reference signs and descriptions thereof will not be repeated.

(Target liquid purification method)

According to the target liquid purification apparatus of the fourth embodiment with the configuration described earlier, a target liquid purification method is performed by the following procedures. Each procedure or each step may be executed in parallel. The unpurified target liquid F11 is introduced into the first portion 110 of the separation apparatus 100 (S1). The filtration dialysis effluent F21 is generated by introducing the unpurified target liquid F11 containing the target substance from the first portion 110 into the second portion 120 in the separation apparatus 100 by passing the unpurified target liquid F11 through the porous membrane 102 (S2). The purified target liquid F12 is generated by removing the target substance contained in the unpurified target liquid F11 by passing through the porous membrane 102 (S3). The purified target liquid F12 is guided out from the first portion 110 of the separation apparatus 100 (S4). The filtration dialysis effluent F21 is guided out from the second portion 120 of the separation apparatus 100 (S5). Among the first liquid F211 and the second liquid F212 as a result of the filtration dialysis effluent F21 being separated, the first liquid F211 is discarded as an effluent (S6). Due to the second liquid F212 coming into contact with the adsorbent in the regenerating unit 200, at least a part of the target substance is removed from the second liquid F212 and the regenerated liquid F22 with a reduced concentration of the target substance is generated (S7). The regenerated liquid F22 is introduced into the second portion 120 of the separation apparatus 100 (S8). Then, the first replenishment liquid F41 is directly mixed with the unpurified target liquid F11 (S9').

(Fifth embodiment)

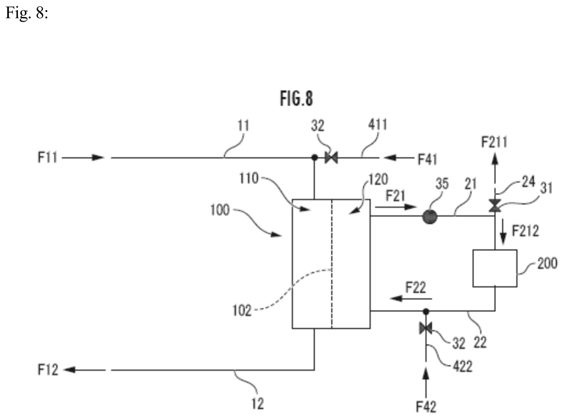

A target liquid purification apparatus as a fifth embodiment shown in FIG. 8 has a configuration in which a second replenishment liquid passage 422 has been added to the target liquid purification apparatus (refer to FIG. 7) according to the fourth embodiment. The second replenishment liquid passage 422 is configured to directly introduce a second replenishment liquid F42 into the regenerated liquid passage 22. Supply sources of the second replenishment liquid F42 and the first replenishment liquid F41 may be a common supply source or separate supply sources.

Since other components of the target liquid purification apparatus according to the fifth embodiment are substantially similar to those of the target liquid purification apparatus according to the fourth embodiment, similar components will be assigned the same reference signs and descriptions thereof will not be repeated.

Since other components of the replenishment liquid flow rate adjustment method according to the fifth embodiment are substantially similar to those of the target liquid purification method according to the second embodiment or the target liquid purification method according to the fourth embodiment, similar components will be assigned the same reference signs and descriptions thereof will not be repeated.

(Sixth embodiment)

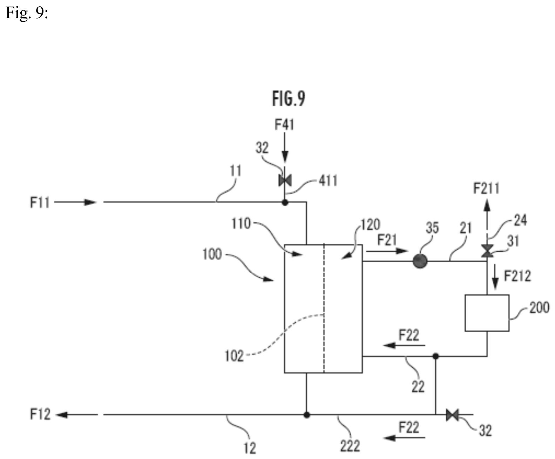

A target liquid purification apparatus as a sixth embodiment shown in FIG. 9 has a configuration in which a regenerated liquid branch passage 222 has been added to the target liquid purification apparatus (refer to FIG. 7) according to the fourth embodiment. The regenerated liquid branch passage 222 is configured to directly introduce a part of the regenerated liquid F22 into the purified target liquid passage 12.

Since other components of the target liquid purification apparatus according to the sixth embodiment are substantially similar to those of the target liquid purification apparatus according to the fourth embodiment, similar components will be assigned the same reference signs and descriptions thereof will not be repeated.

Since other components of the replenishment liquid flow rate adjustment method according to the sixth embodiment are substantially similar to those of the target liquid purification method according to the second embodiment or the target liquid purification method according to the fourth embodiment, similar components will be assigned the same reference signs and descriptions thereof will not be repeated.

(Effects)

According to the target liquid purification apparatuses and the target liquid purification methods of the embodiments described above, the replenishment liquid is supplied to an appropriate location in the regenerating circuit from the perspective of reducing a total amount of dialysate used in the dialysis method. Reducing the transport amount of dialysate enables the apparatus to be downsized, resulting in configurations suitable for home use as well as environmentally-conscious configurations.

(Other embodiments)

The first replenishment liquid passage 411 in the target liquid purification apparatuses (refer to FIGS. 7 to 9) according to the fourth to sixth embodiments may be added to the target liquid purification apparatuses (refer to FIG. 1 and FIGS. 5 and 6) according to the first to third embodiments. In this case, the first replenishment liquid is directly supplied to or introduced into each of the unpurified target liquid passage 11 and the purified target liquid passage 12 from each of the pair of first replenishment liquid passages 411 and 412. Each of the pair of first replenishment liquid passages 411 and 412 may be communicated with a common replenishment liquid supply source or communicated with separate replenishment liquid supply sources.

The regenerated liquid branch passage 222 according to the sixth embodiment (refer to FIG. 9) may be added to the target liquid purification apparatuses (refer to FIG. 1 and FIGS. 5 to 7) according to the first to fifth embodiments. The regenerated liquid branch passage 221 according to the third embodiment (refer to FIG. 6) may be added to the target liquid purification apparatuses (refer to FIG. 5 and FIGS. 7 to 9) according to the second and fourth to sixth embodiments.

(Abnormality detection method)

In addition to components of the embodiments described above, the target liquid purification apparatus comprises: the abnormality detection apparatus 301 configured to detect an abnormality based on a time-series variation in a concentration of a target substance; and the notification apparatus 400 configured to notify an abnormality detected by the abnormality detection apparatus 301.

The abnormality detection apparatus 301 is an apparatus which constitutes a part of the control apparatus 300 and which detects an abnormality based on a time series of the concentration of the target substance. In this case, a time-series variation in the concentration of the target substance refers to, for example, information indicating the value of the first concentration $C_1^{\rightarrow}$ described earlier for each time series. In this case, values of a plurality of target substances may be adopted as values of the first concentration $C_1^{\rightarrow}$. Based on time-series information of the concentrations of the plurality of target substances, an abnormal value is calculated statistically or using a method of machine learning. For example, when the value of the first concentration $C_1^{\rightarrow}$ does not vary, it can be determined that dialysis is not performed sufficiently. In addition, when a specific substance is present in a larger amount than other substances, it can be determined that the composition of the replenishment liquid is insufficient.

The notification apparatus 400 is connected to the target liquid purification apparatus via wireless communication such as a network or via wired communication such as a cable (refer to FIG. 1). The notification apparatus 400 is, for example, software installed on a personal computer, a smartphone, or the like. The notification apparatus 400 is carried by, for example, a patient using the target liquid purification apparatus, their family members, their assigned healthcare professionals, or the like.

When the abnormality detection apparatus 301 detects an abnormality, the detection of the abnormality is notified to the output interface 402 of the notification apparatus 400. Accordingly, a user can recognize deterioration of the target liquid purification apparatus or the like and, based on the recognition, reliably execute purification of the target liquid. Furthermore, since healthcare professionals can recognize the presence or absence of disease, disease risk factors, or the like in advance, the healthcare professionals can effectively perform target liquid purification to maintain the health of patients using the target liquid purification apparatus.

Example

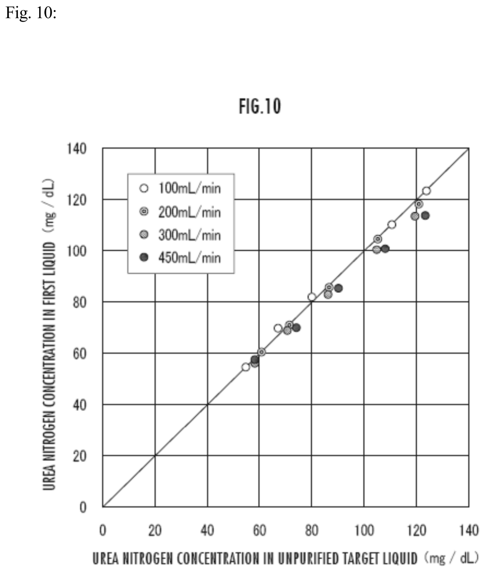

A first example is a target liquid purification apparatus with the configuration of the first embodiment. FIG. 10 is a diagram showing a concentration of urea nitrogen as a target substance in the unpurified target liquid F11 and a concentration of urea nitrogen in the first liquid F211 when the flow rate of the replenishment liquid F41 is set to 100, 200, 300, and 450 mL/min in the target liquid purification apparatus according to the first example. Note that, here, the unpurified target liquid F11 (blood flow rate) is 250 mL/min. Referring to FIG. 10, when the flow rate of the replenishment liquid F41 was greater than the flow rate of the unpurified target liquid F11 (replenishment liquid F41 flow rate: 100, 200 mL/min), the concentration of urea nitrogen in the unpurified target liquid F11 was approximately the same as the concentration of urea nitrogen in the first liquid F211. On the other hand, when the flow rate of the replenishment liquid F41 was greater than the flow rate of the unpurified target liquid F11 (replenishment liquid F41 flow rate: 300, 450 mL/min), the concentration of urea nitrogen in the first liquid F211 was lower than the concentration of urea nitrogen in the unpurified target liquid F11.

Here, when a removal performance achieved after a single pass through the target liquid purification apparatus is expressed by a clearance CL index, the clearance CL is expressed by the following equation (1), using a urea nitrogen concentration $C_{BI}$ in the unpurified target liquid F11, a urea nitrogen concentration $C_{BO}$ in the purified target liquid F12, and a flow rate $Q_B$ of the unpurified target liquid F11.

$$CL = ((C_{BI} - C_{BO})/C_{BI}) * Q_B \dots (1)$$

Here, CL being 230 to 250 mL/min when $Q_B$ is 250 mL/min means that approximately 100% of urea nitrogen is removed in a single pass of the target liquid purification apparatus. In addition, based on material balance, a concentration of urea nitrogen in the first liquid F211 at this time is expressed by the following equation (2), using a concentration of urea nitrogen in the replenishment liquid F41, a concentration of urea nitrogen in the first liquid F211, and a flow rate of the replenishment liquid F41 as $C_{DI}$, $C_{DO}$, and $Q_D$, respectively.

$$Q_B*(C_{BI} - C_{BO}) = Q_D*(C_{DO} - C_{DI}) \dots (2)$$

Furthermore, using an overall mass transfer area coefficient KoA yields the following relational expression (3).

$$(C_{BI} - C_{BO})/(C_{BI} - C_{DI}) =$$

$$(1 - \exp(KoA(1/Q_{BI} - 1/Q_{BO})))/(Q_{BI}/Q_{DI} - \exp(KoA(1/Q_{BI} -1/Q_{BO}))) \dots (3)$$

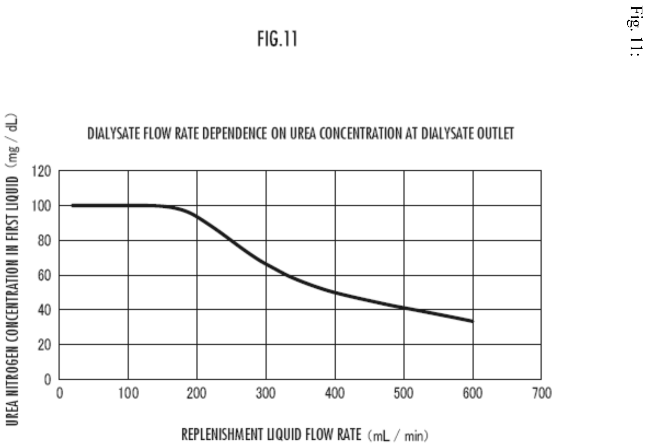

$C_{DO}$ can be determined based on the above relational expressions (1) to (3). At this point, setting operating conditions to $Q_B$ = 200 mL/min and $C_{BI}$ = 100 mg/dL, since in the case for urea nitrogen, KoA can be considered to be around 3000, setting a length of the separation apparatus (dialyzer) to 25 cm and a membrane area to 1.5 m$^2$ enables $C_{DO}$ when $Q_D$ is varied to be calculated, yielding FIG. 11.

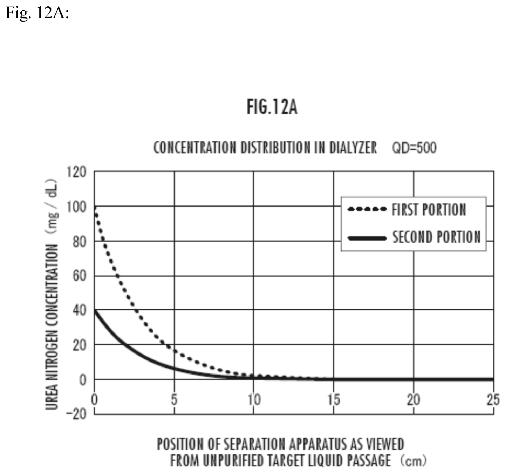

Referring to FIG. 11, when the flow rate of the replenishment liquid F41 becomes smaller than approximately 200 mL/min, which is approximately equal to the flow rate of the unpurified target liquid F11, the first liquid F211 has a concentration of approximately 100 mg/dL, which is approximately equal to that of the unpurified target liquid F11. This is attributable to an extremely high transfer rate of urea nitrogen from the unpurified target liquid F11 to the separation apparatus in the target liquid purification apparatus. At this point, the concentration distribution of urea nitrogen in the unpurified target liquid F11 and in the replenishment liquid F41 within the target liquid purification apparatus can be calculated as shown in FIGs. 12A to 12C.

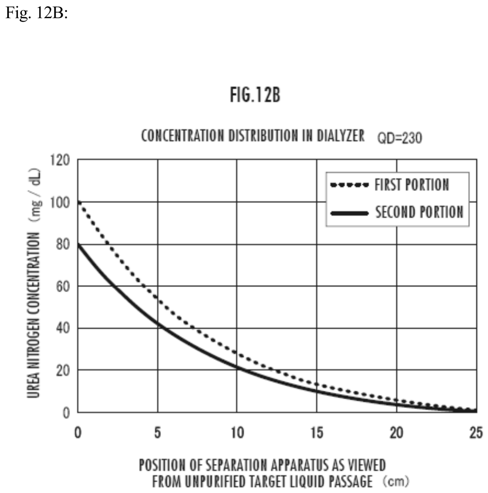

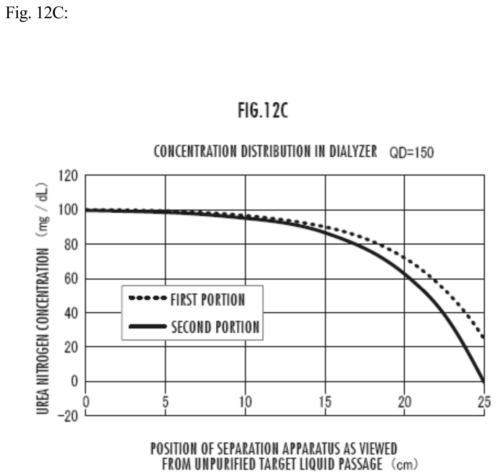

In FIGs. 12A to 12C, an axis of abscissa represents a position in a length direction of the separation apparatus 100 (where an inflow portion of the unpurified target liquid F11 into the separation apparatus is 0 and an outflow portion to the purified target liquid F12 is 25 on the axis of abscissa), and an axis of ordinate represents urea nitrogen concentration. Here, solid lines represent the urea nitrogen concentration in the first portion 110 side and dotted lines represent the urea nitrogen concentration in the second portion 120 side. Referring to FIG. 12A, when the flow rate of the replenishment liquid F41 is 500 mL/min, conceivably, approximately 100% of the urea nitrogen in the unpurified target liquid F11 is being removed (a value of urea nitrogen at 25 cm is approximately zero). Referring to FIG. 12B, when the flow rate of the replenishment liquid F41 is 230 mL/min, approximately 100% of the urea nitrogen is similarly being removed. At this point, a y-intercept value representing the urea nitrogen concentration of the first liquid F211 is 40 mg/dL when the flow rate of the replenishment liquid F41 is 500 mL/min, which is lower than the urea nitrogen concentration of the unpurified target liquid F11. On the other hand, as the flow rate of the replenishment liquid F41 decreases, the urea nitrogen concentration of the first liquid F211 increases. Referring to FIG. 12C, in the graph showing a flow rate of 150 mL/min for the replenishment liquid F41, the urea nitrogen concentration in the unpurified target liquid F11 and the urea nitrogen concentration in the first liquid F211 are each approximately 100 mg/dL and can be considered equivalent. Accordingly, even without having to provide the measurement apparatus 35 in the unpurified target liquid F11 (blood), the urea nitrogen concentration in the unpurified target liquid F11 can be determined by measuring the concentration of urea nitrogen in either the first liquid F211 or the filtration dialysis effluent F21. Accordingly, since the processing described earlier is executed, a total amount of the replenishment liquid F41 in the target liquid purification apparatus can be reduced and, at the same time, purification can be reliably executed.

While the results have been presented using values of urea nitrogen in the example, similar results were obtained with substances other than urea nitrogen that exhibit high permeability through the separation membrane. Although not specifically illustrated, similar results were obtained using potassium ions. Therefore, such a target liquid purification apparatus can accurately measure the concentrations of a plurality of target substances, enabling overall control of the target liquid purification apparatus based on the concentrations.

Based on the above, the target liquid purification apparatus enables control that reliably processes a target substance from a target liquid while reducing the amount of dialysate. Therefore, the target liquid purification apparatus can be used in home dialysis and the like. In addition, if an abnormality occurs during home dialysis, since the abnormality can be transmitted to remote medical professionals, reliable dialysis treatment can be provided.

It should be noted that the present invention is not limited to the embodiments or the examples described above, and the scope of the present invention can clearly be modified or altered within the scope obvious to those skilled in the art. Therefore, it should be obvious that the scope of the present invention is not limited to the embodiments or the examples described above but also includes modifications and alterations thereof.

Reference Signs List

| 11 | unpurified target liquid passage |
|---|---|
| 12 | purified target liquid passage |
| 21 | filtration dialysis effluent passage |
| 22 | regenerated liquid passage |
| 24 | effluent passage |
| 31 | first flow rate adjustment apparatus |
| 32 | second flow rate adjustment apparatus |
| 35 | measurement apparatus |
| 100 | separation apparatus |
| 102 | porous membrane |
| 110 | first portion |
| 120 | second portion |
| 200 | regenerating unit |
| 201 | regenerating circuit |
| 202 | regenerating circuit |
| 221 | regenerated liquid branch passage |
| 222 | regenerated liquid branch passage |
| 300 | control apparatus |
| 301 | abnormality detection apparatus |
| 400 | notification apparatus |
| 401 | input interface |
| 402 | output interface |

411     first replenishment liquid passage
412     first replenishment liquid passage
422     second replenishment liquid passage
2011    first circuit (regenerating circuit 201)
2012    second circuit (regenerating circuit 201)
2013    third flow rate adjustment apparatus (regenerating circuit 201)
2021    first circuit (regenerating circuit 202)
2022    second circuit (regenerating circuit 202)
2023    third flow rate adjustment apparatus (regenerating circuit 202)
F11     unpurified target liquid
F12     purified target liquid
F21     filtration dialysis effluent
F211    first liquid (effluent)
F212    second liquid
F22     regenerated liquid
F41     first replenishment liquid
F42     second replenishment liquid.

**Claims**

1. A target liquid purification apparatus that removes at least one of a plurality of target substances from an unpurified target liquid, the target liquid purification apparatus comprising:

   a separation apparatus including a porous membrane having a pore size through which the target substances contained in the unpurified target liquid can pass and a first portion and a second portion separated by the porous membrane, the separation apparatus being configured to generate a filtration dialysis effluent by introducing the unpurified target liquid containing the target substances from the first portion into the second portion while passing the unpurified target liquid through the porous membrane and to generate a purified target liquid by removing the target substances contained in the unpurified target liquid by the pass-through;
   an unpurified target liquid passage configured to introduce the unpurified target liquid into the first portion of the separation apparatus;
   a purified target liquid passage configured to guide the purified target liquid out from the first portion of the separation apparatus;
   a filtration dialysis effluent passage configured to guide the filtration dialysis effluent out from the second portion of the separation apparatus;
   an effluent passage which branches from the filtration dialysis effluent passage and which is configured to discard, among a first liquid and a second liquid as a result of the filtration dialysis effluent being separated, the first liquid as an effluent;
   a regenerating unit which includes an adsorbent configured to adsorb the target substances, which is configured to remove at least one of the target substances from the second liquid by bringing the second liquid having been introduced from the filtration dialysis effluent passage in contact with the adsorbent and to generate a regenerated liquid with a reduced concentration of the target substances, and which includes two or more regenerating circuits connected in series;
   a regenerated liquid passage configured to introduce the regenerated liquid from the regenerating unit into the second portion of the separation apparatus;
   a first replenishment liquid passage configured to directly introduce a replenishment liquid into at least one of the unpurified target liquid passage and the purified target liquid passage; and
   at least one measurement apparatus which is provided in any of the unpurified target liquid passage, the separation apparatus, and the filtration dialysis effluent passage and which is configured to measure a concentration of the target substances.

2. The target liquid purification apparatus according to claim 1, comprising

   a first circuit on which one of the regenerating circuits brings an upstream-side liquid into contact with the adsorbent and then guides the liquid out to the downstream side; a second circuit on which one of the regenerating circuits introduces an upstream-side liquid into the downstream side without bringing the liquid into contact with the adsorbent; and a flow rate adjustment apparatus configured to adjust respective flow rates of the upstream-side liquid introduced into the first circuit and the second circuit, wherein

the flow rate adjustment apparatus is configured to adjust the flow rate introduced into the first circuit and the flow rate introduced into the second circuit according to a concentration of the target substances in the filtration dialysis effluent measured by the measurement apparatus.

3. The target liquid purification apparatus according to claim 1 or 2, comprising:

an abnormality detection apparatus configured to detect an abnormality based on a time-series variation in the concentration of the target substances; and
a notification apparatus configured to notify an abnormality detected by the abnormality detection apparatus.

Fig. 1:

FIG.1

Fig. 2:

FIG.2

Fig. 3:

FIG.3

START

S310 — $OP_1 \leftarrow OP_{10}$, $OP_2 \leftarrow OP_{20}$, $f \leftarrow 0$

$X_0$

S311 — $C_1 \leftarrow C$, $t \leftarrow 0$

$X_1$

S312 — $C_1 \leq C_0$ — YES / NO

S313 — $f \leftarrow f + 1$

S314 — $f \geq f_0$ — NO → $X_1$ / YES → END

END

S315 — $t \leftarrow t + 1$

S316 — $t \geq t_0$ — NO / YES

S317 — $C_2 \leftarrow C$

S318 — $C_1 - C_2 \leq \Delta C_1$ — YES / NO

S319 — REDUCE FLOW RATE OF AT LEAST ONE OF F11, F21, AND F41 → $X_0$

S320 — $C_1 - C_2 \geq \Delta C_2$ — YES / NO

S321 — INCREASE FLOW RATE OF AT LEAST ONE OF F11, F21, AND F41 → $X_0$

EP 4 744 691 A1

FIG.4

START

$X_0$

S410
$OP_3 \leftarrow OP_{30}, f_1 \leftarrow 0$

S411
$C_{11} \leftarrow C_{10}, t \leftarrow 0$

S412
$X_1$   $C_{11} \leqq C_{100}$   YES

NO

S413
$f \leftarrow f + 1$

S414
$f_1 \geqq f_{10}$   NO

YES

S422
$OP_3 \leftarrow 0$

S415
$t \leftarrow t + 1$

S416
$t \geqq t_{10}$   NO

YES

S417
$C_{21} \leftarrow C_{10}$

S418
NO   $C_{11} - C_{21} \leqq \Delta C_{11}$

YES

S419
$OP_3 \leftarrow OP_3 - \Delta OP_{31}$

S420
NO   $C_{11} - C_{21} \geqq \Delta C_{21}$

YES

S421
$OP_3 \leftarrow OP_3 + \Delta OP_{32}$

$X_0$

$X_0$

$X_1$

EP 4 744 691 A1
Fig. 5:

24

Fig. 6:

FIG.6

Fig. 7:

FIG.7

Fig. 8:

FIG.8

Fig. 9:

FIG.9

Fig. 10:

FIG.10

Fig. 11:

FIG.11

DIALYSATE FLOW RATE DEPENDENCE ON UREA CONCENTRATION AT DIALYSATE OUTLET

Fig. 12A:

## FIG.12A

CONCENTRATION DISTRIBUTION IN DIALYZER  QD=500

Fig. 12B:

**FIG.12B**

CONCENTRATION DISTRIBUTION IN DIALYZER  QD=230

Fig. 12C:

## FIG.12C

CONCENTRATION DISTRIBUTION IN DIALYZER   QD=150

POSITION OF SEPARATION APPARATUS AS VIEWED
FROM UNPURIFIED TARGET LIQUID PASSAGE （cm）

**EP 4 744 691 A1**

## INTERNATIONAL SEARCH REPORT

International application No.

**PCT/JP2025/001917**

### A. CLASSIFICATION OF SUBJECT MATTER

*A61M 1/16*(2006.01)i; *A61M 1/34*(2006.01)i
FI: A61M1/16 190; A61M1/34 120; A61M1/16 111; A61M1/16 117

According to International Patent Classification (IPC) or to both national classification and IPC

### B. FIELDS SEARCHED

Minimum documentation searched (classification system followed by classification symbols)

A61M1/00-1/38

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 10-71201 A (TERUMO KABUSHIKI KAISHA) 17 March 1998 (1998-03-17) paragraphs [0001], [0035]-[0052], fig. 2 | 1 |
| Y | | 3 |
| A | | 2 |
| Y | JP 62-7863 B2 (YUNO RABO KK) 19 February 1987 (1987-02-19) column 5, lines 2-6, fig. 3 | 3 |
| A | JP 5992453 B2 (FRESENIUS MEDICAL CARE HOLDINGS, INC.) 14 September 2016 (2016-09-14) paragraphs [0150]-[0152], fig. 26-27 | 1-3 |

☐ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **03 March 2025** | **25 March 2025** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (July 2022)

34

**INTERNATIONAL SEARCH REPORT**
Information on patent family members

International application No.

**PCT/JP2025/001917**

| Patent document cited in search report | | | Publication date (day/month/year) | Patent family member(s) | | | Publication date (day/month/year) |
|---|---|---|---|---|---|---|---|
| JP | 10-71201 | A | 17 March 1998 | (Family: none) | | | |
| JP | 62-7863 | B2 | 19 February 1987 | JP | 58-69571 | A | |
| JP | 5992453 | B2 | 14 September 2016 | WO | 2012/108910 | A1 | |
| | | | | description, p. 49, lines 3-26, fig. 26-27 | | | |
| | | | | AU | 2009206044 | A | |
| | | | | AU | 2009302327 | A | |
| | | | | AU | 2009320007 | A1 | |
| | | | | AU | 2010203362 | A1 | |
| | | | | AU | 2011358554 | A1 | |
| | | | | AU | 2013370583 | A1 | |
| | | | | AU | 2014240817 | A1 | |
| | | | | AU | 2014262300 | A1 | |
| | | | | AU | 2015202780 | A1 | |
| | | | | AU | 2017203445 | A1 | |
| | | | | AU | 2018201518 | A1 | |
| | | | | AU | 2019271940 | A | |
| | | | | BR | 112013020260 | A2 | |
| | | | | BR | 112015015234 | A2 | |
| | | | | BR | 112015024862 | A2 | |
| | | | | BR | PI0921637 | A2 | |
| | | | | CA | 2706919 | A1 | |
| | | | | CA | 2712461 | A1 | |
| | | | | CA | 2739786 | A1 | |
| | | | | CA | 2739807 | A1 | |
| | | | | CA | 2749171 | A1 | |
| | | | | CA | 2826775 | A1 | |
| | | | | CA | 2894387 | A1 | |
| | | | | CA | 2907489 | A1 | |
| | | | | CA | 2928208 | A1 | |
| | | | | CA | 2960103 | A1 | |
| | | | | CA | 2976872 | A1 | |
| | | | | CA | 3002932 | A1 | |
| | | | | CA | 3057806 | A1 | |
| | | | | CA | 3057807 | A1 | |
| | | | | CN | 102046260 | A | |
| | | | | CN | 102271753 | A | |
| | | | | CN | 102307650 | A | |
| | | | | CN | 102639201 | A | |
| | | | | CN | 103476486 | A | |
| | | | | CN | 104307210 | A | |
| | | | | CN | 105056324 | A | |
| | | | | CN | 105120914 | A | |
| | | | | CN | 105148344 | A | |
| | | | | CN | 105263542 | A | |
| | | | | CN | 107376045 | A | |
| | | | | CN | 107441575 | A | |
| | | | | CN | 109939283 | A | |
| | | | | EA | 201170628 | A1 | |
| | | | | EA | 201170924 | A1 | |

Form PCT/ISA/210 (patent family annex) (July 2022)

## INTERNATIONAL SEARCH REPORT
### Information on patent family members

International application No.

**PCT/JP2025/001917**

| Patent document cited in search report | Publication date (day/month/year) | Patent family member(s) | | | Publication date (day/month/year) |
|---|---|---|---|---|---|
| | | EA | 201690595 | A1 | |
| | | EP | 2237814 | A1 | |
| | | EP | 2237851 | A1 | |
| | | EP | 2334412 | A2 | |
| | | EP | 2342003 | A2 | |
| | | EP | 2379922 | A1 | |
| | | EP | 2673073 | A1 | |
| | | EP | 2934621 | A1 | |
| | | EP | 2999497 | A1 | |
| | | EP | 3511034 | A1 | |
| | | EP | 3520837 | A1 | |
| | | EP | 3524286 | A1 | |
| | | EP | 3586946 | A1 | |
| | | EP | 3741402 | A1 | |
| | | HK | 1161152 | A1 | |
| | | HK | 1165749 | A1 | |
| | | HK | 1173693 | A1 | |
| | | HK | 1203437 | A1 | |
| | | HK | 1218084 | A1 | |
| | | HK | 1218890 | A1 | |
| | | HK | 1220417 | A1 | |
| | | JP | 2011-509760 | A | |
| | | JP | 2012-510826 | A | |
| | | JP | 2012-515305 | A | |
| | | JP | 2013-176682 | A | |
| | | JP | 2014-516255 | A | |
| | | JP | 2015-42266 | A | |
| | | JP | 2016-504111 | A | |
| | | JP | 2016-517719 | A | |
| | | JP | 2017-29734 | A | |
| | | JP | 2017-60802 | A | |
| | | JP | 2017-196446 | A | |
| | | JP | 2019-48098 | A | |
| | | JP | 2019-103824 | A | |
| | | JP | 2020-36901 | A | |
| | | KR | 10-2011-0090912 | A | |
| | | KR | 10-2014-0024853 | A | |
| | | KR | 10-2015-0102057 | A | |
| | | KR | 10-2016-0047428 | A | |
| | | MX | 2010005907 | A | |
| | | MX | 2010007856 | A | |
| | | MX | 2011003737 | A | |
| | | MX | 2011004600 | A | |
| | | MX | 2011007443 | A | |
| | | MX | 2013009059 | A | |
| | | MX | 2015007314 | A | |
| | | MX | 2015013489 | A | |
| | | MX | 340061 | B | |
| | | MX | 347636 | B | |
| | | NZ | 586924 | A | |

Form PCT/ISA/210 (patent family annex) (July 2022)

## INTERNATIONAL SEARCH REPORT
### Information on patent family members

International application No.

**PCT/JP2025/001917**

| Patent document cited in search report | Publication date (day/month/year) | Patent family member(s) | | | Publication date (day/month/year) |
|---|---|---|---|---|---|
| | | NZ | 592652 | A | |
| | | NZ | 592653 | A | |
| | | NZ | 601028 | A | |
| | | NZ | 614023 | A | |
| | | NZ | 614053 | A | |
| | | NZ | 627386 | A | |
| | | RU | 2013137763 | A | |
| | | RU | 2015122432 | A | |
| | | RU | 2015139684 | A | |
| | | US | 2016/0109429 | A1 | |
| | | US | 2016/0317733 | A1 | |
| | | US | 2016/0319954 | A1 | |
| | | US | 2017/0007756 | A1 | |
| | | US | 2017/0021085 | A1 | |
| | | US | 2017/0021088 | A1 | |
| | | US | 2017/0021306 | A1 | |
| | | US | 2017/0232177 | A1 | |
| | | US | 2018/0017542 | A1 | |
| | | US | 2018/0339269 | A1 | |
| | | US | 2018/0361049 | A1 | |
| | | US | 2019/0111201 | A1 | |
| | | US | 2019/0134566 | A1 | |
| | | US | 2019/0203848 | A1 | |
| | | US | 2020/0061280 | A1 | |
| | | US | 2020/0155756 | A1 | |
| | | US | 2020/0254169 | A1 | |
| | | US | 2020/0326324 | A1 | |
| | | US | 2020/0397971 | A1 | |
| | | US | 2020/0406193 | A1 | |
| | | US | 2021/0001033 | A1 | |
| | | US | 2009/0076434 | A1 | |
| | | US | 2009/0101552 | A1 | |
| | | US | 2009/0101577 | A1 | |
| | | US | 2009/0114037 | A1 | |
| | | US | 2009/0120864 | A1 | |
| | | US | 2009/0173682 | A1 | |
| | | US | 2009/0282980 | A1 | |
| | | US | 2010/0116048 | A1 | |
| | | US | 2010/0116740 | A1 | |
| | | US | 2010/0140149 | A1 | |
| | | US | 2010/0179464 | A1 | |
| | | US | 2010/0184198 | A1 | |
| | | US | 2010/0234786 | A1 | |
| | | US | 2010/0252490 | A1 | |
| | | US | 2010/0331754 | A1 | |
| | | US | 2011/0054378 | A1 | |
| | | US | 2011/0315611 | A1 | |
| | | US | 2012/0031825 | A1 | |
| | | US | 2012/0073365 | A1 | |
| | | US | 2012/0090706 | A1 | |

Form PCT/ISA/210 (patent family annex) (July 2022)

## INTERNATIONAL SEARCH REPORT
### Information on patent family members

International application No.

**PCT/JP2025/001917**

| Patent document cited in search report | Publication date (day/month/year) | Patent family member(s) | | Publication date (day/month/year) |
|---|---|---|---|---|
| | | US | 2012/0103885 A1 | |
| | | US | 2012/0204968 A1 | |
| | | US | 2012/0280154 A1 | |
| | | US | 2013/0220907 A1 | |
| | | US | 2013/0292319 A1 | |
| | | US | 2014/0138294 A1 | |
| | | US | 2014/0339149 A1 | |
| | | WO | 2009/073567 A1 | |
| | | WO | 2009/091963 A1 | |
| | | WO | 2010/081121 A1 | |
| | | WO | 2010/114932 A1 | |
| | | WO | 2014/105755 A1 | |
| | | WO | 2014/161008 A1 | |
| | | WO | 2010/042666 A2 | |
| | | WO | 2010/042667 A2 | |
| | | WO | 2010/062698 A2 | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022132837 A

- US 9302038 B

**Non-patent literature cited in the description**

- **STEVEN M. BRUNELLI** ; **DAVID M. SPIEGEL** ; **CHARLES DU MOND** ; **NINA OESTREICHER** ; **WOLFGANG C. WINKELMAYER** ; **CSABA P. KOVESDY**. Serum-to-dialysate potassium gradient and its association with short-term outcomes in hemodialysis patients. *Nephrol Dial Transplant*, 2018, vol. 33, 1207-1214

- **REDAELLI B** ; **LOCATELLI F** ; **LIMIDO D et al.** Effect of a new model of hemodialysis potassium removal on the control of ventricular arrhythmias. *Kidney Int*, 1996, vol. 50, 609-617
- *Journal of the Japanese Society for Dialysis Therapy*, 2013, vol. 43 (7), 587-632